# EUROPÄISCHE PATENTSCHRIFT

(11) **EP 2 507 367 B1**
(45) Veröffentlichungstag und Bekanntmachung des Hinweises auf die Patenterteilung: **23.11.2016**
(21) Anmeldenummer: 10784539.8
(22) Anmeldetag: 30.11.2010
(51) Int. Cl.: C12N 9/04, C12P 33/02, C12P 33/06

(54) **NEUARTIGE 7 -HYDROXYSTEROID DEHYDROGENASEN UND DEREN VERWENDUNG**
NOVEL 7 -HYDROXYSTEROID DEHYDROGENASES AND THEIR USE
NOUVELLES 7 -HYDROXYSTÉROÏDES DÉSHYDROGÉNASES ET LEUR UTILISATION

(30) Priorität: 30.11.2009 EP 09177544; 25.08.2010 EP 10008837
(43) Veröffentlichungstag der Anmeldung: 10.10.2012
(73) Patentinhaber: PharmaZell GmbH, 83064 Raubling (DE)
(72) Erfinder: LIU, Luo, Beijing 102445 (CN); SCHMID, Rolf, 70569 Stuttgart (DE); AIGNER, Arno, 83104 Tuntenhausen (DE)
(74) Vertreter: Reitstötter Kinzebach
(86) Internationale Anmeldenummer: PCT/EP2010/068576
(87) Internationale Veröffentlichungsnummer: WO 2011/064404

(56) Entgegenhaltungen:
- WO-A2-2009/118176
- DATABASE UniProt [Online] 17. April 2007 (2007-04-17), Sudarsanam P. et al.: "SubName: Full=Putative uncharacterized protein", XP002579158, Database accession no. A4ECA9
- HIRANO S ET AL: "Characterization of NADP-dependent 7 beta-hydroxysteroid dehydrogenases from Peptostreptococcus productus and Eubacterium aerofaciens.", Mai 1982 (1982-05), APPLIED AND ENVIRONMENTAL MICROBIOLOGY MAY 1982 LNKD- PUBMED:6954878, VOL. 43, NR. 5, PAGE(S) 1057 - 1063, XP002579159, ISSN: 0099-2240 das ganze Dokument
- AKAO T ET AL: "Purification and characterization of 7 beta-hydroxysteroid dehydrogenase from Ruminococcus sp. of human intestine", JOURNAL OF BIOCHEMISTRY, JAPANESE BIOCHEMICAL SOCIETY / OUP, TOKYO; JP, Bd. 102, Nr. 3, 1. Januar 1987 (1987-01-01), Seiten 613-619, XP008135698, ISSN: 0021-924X
- CARREA GIACOMO ET AL: "Enzymatic synthesis of 12-ketoursodeoxycholic acid from dehydrocholic acid in a membrane reactor", 1992, BIOTECHNOLOGY LETTERS, VOL. 14, NR. 12, PAGE(S) 1131-1134, XP002579160, ISSN: 0141-5492 das ganze Dokument
- BOVARA ROBERTO ET AL: "A new enzymatic route to the synthesis of 12-ketoursodeoxycholic acid", 1996, BIOTECHNOLOGY LETTERS, VOL. 18, NR. 3, PAGE(S) 305-308, XP002579162, ISSN: 0141-5492 das ganze Dokument
- LIU LUO ET AL: "Identification, cloning, heterologous expression, and characterization of a NADPH-dependent 7[beta]-hydroxysteroid dehydrogenase from Collinsella aerofaciens.", APPLIED MICROBIOLOGY AND BIOTECHNOLOGY APR 2011 LNKD- PUBMED:21181147, Bd. 90, Nr. 1, April 2011 (2011-04), Seiten 127-135, XP002634164, ISSN: 1432-0614

## Beschreibung

Die Erfindung betrifft neuartige 7ß-Hydroxysteroid Dehydrogenasen erhältlich aus Bakterien der Gattung *Collinsella,* insbesondere des Stammes *Collinsella aerofaciens,* die für diese Enzyme kodierenden Sequenzen, Verfahren zur Herstellung der Enzyme und deren Verwendung bei enzymatischen Umsetzungen von Cholsäureverbindungen, und insbesondere bei der Herstellung von Ursodesoxycholsäure (UDCS); Gegenstand der Erfindung sind auch neuartige Verfahren zur Synthese von UDCS.

### Hintergrund der Erfindung:

Zur medikamentösen Behandlung von Gallensteinleiden werden seit vielen Jahren u. a. die Wirkstoffe Ursodesoxycholsäure (UDCS) und das zugehörige Diastereomer Chenodesoxycholsäure (CDCS) eingesetzt. Beide Verbindungen unterscheiden sich lediglich durch die Konfiguration der Hydroxygruppe am C-Atom 7 (UDCS: β-Konfiguration, CDCS: α-Konfiguration). Zur Herstellung von UDCS sind im Stand der Technik verschieden Verfahren beschreiben, welche rein chemisch durch geführt werden oder aus einer Kombination chemischer und enzymatischer Verfahrensschritte bestehen. Ausgangspunkt ist jeweils Cholsäure (CS) oder die aus Cholsäure hergestellte CDCS.

So kann die klassisch chemische Methode zur UDCS Herstellung wie folgt schematisch dargestellt werden:

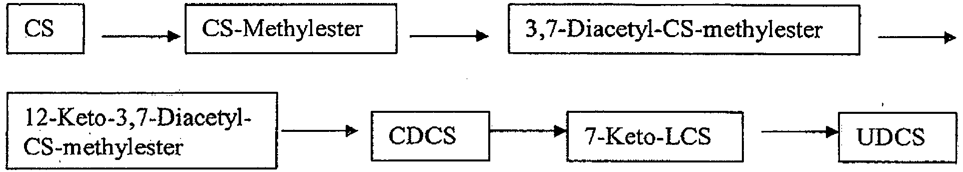

Ein gravierender Nachteil ist unter anderem folgender: da die chemische Oxidation nicht selektiv ist, müssen die Carboxy-Gruppe und die 3α und 7α-Hydroxy-Gruppe durch Veresterung geschützt werden.

Ein alternatives chemisch/enzymatische Verfahren basierend auf der Verwendung des Enzyms 12α-Hydroxysteroid Dehydrogenase (12α-HSDH) kann wie folgt dargestellt werden und ist z.B. beschrieben in der PCT/EP2009/002190 der vorliegenden Anmelderin.

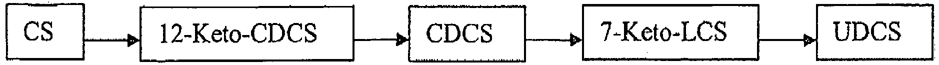

Die 12α-HSDH oxidiert dabei CS selektiv zu 12-Keto-CDCS. Die zwei nach der klassisch chemischen Methode erforderlichen Schützschritte entfallen dabei.

Weiterhin wird von Monti, D., et al., (One-Pot Multienzymatic Synthesis of 12-Ketoursodeoxycholic Acid: Subtle Cofactor Specificities Rule the Reaction Equilibria of Five Biocatalysts Working in a Row. Advanced Synthesis & Catalysis, 2009) ein alternatives enzymatisch-chemisches Verfahren beschrieben, das wie folgt schematisch darstellbar ist:

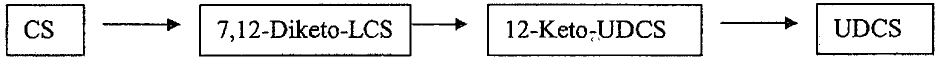

Die CS wird zuerst von 7α-HSDH aus *Bacteroides fragilis* ATCC 25285 (Zhu, D., et al., Enzymafic enantioselective reduction of -ketoesters by a thermostable 7 -hydroxysteroid dehydrogenase from Bacteroides fragilis. Tetrahedron, 2006. 62(18): p. 4535-4539) und 12α-HSDH zu 7,12-Diketo-LCS oxidiert. Diese beiden Enzyme sind jeweils NADH abhängig. Nach der Reduktion durch 7β-HSDH (NADPH abhängig) aus *Clostridium absonum* ATCC 27555 (DSM 599) (MacDonald, I.A. and P.D. Roach, Bile induction of 7 alpha- and 7 beta-hydroxysteroid dehydrogenases in Clostridium absonum. Biochim Biophys Acta, 1981. 665(2): p. 262-9) entsteht 12-Keto-UDCS. Durch Wolff-Kishner-Reduktion wird das Endprodukt erhalten. Nachteilig bei diesem Verfahren ist, dass wegen der Gleichgewichtslage der katalysieren Reaktion eine komplette Umsetzung nicht möglich ist, und dass für die erste Stufe der Umsetzung zwei unterschiedliche Enzyme eingesetzt werden müssen, was das Verfahren verteuert. Zur Kofaktor-Regenerierung werden Lactat Dehydrogenase (LDH; zur Regenerierung von NAD⁺) und Glucose Dehydrogenase (GlcDH, zur Regenerierung von NADPH) eingesetzt. Nachteilig bei der dort verwendeten Kofaktor-Regenerierung ist, dass das entstehende Ko-Produkt nur sehr schwer aus dem Reaktionsgemisch zu entfernen ist, so dass das Reaktionsgleichgewicht nicht positiv beeinflusst werden kann, was eine unvollständige Umsetzung des Edukts bedingt.

Eine 7β-HSDH aus Stamm *Collinsella aerofaciens* ATCC 25986 (DSM 3979; ehemalige *Eubacterium aerofaciens*) wurde im Jahr 1982 von Hirano und Masuda beschrieben (Hirano, S. and N. Masuda, Characterization of NADP-dependent 7 beta-hydroxysteroid dehydrogenases from Peptostreptococcus productus and Eubacterium aerofaciens. Appl Environ Microbiol, 1982. 43(5): p. 1057-63). Sequenzinformationen wurden zu diesem Enzym nicht offenbart. Das durch Gelfiltration bestimmte Molekulargewicht betrug 45,000 Da (vgl. Hirano, Seite 1059, linke Spalte). Weiterhin konnte für das dortige Enzym die Reduktion der 7-Oxo-Gruppe zur 7β-Hydroxygruppe nicht beobachtet werden (vgl. Hirano, Seite 1061, Diskussion 1. Absatz). Entsprechend werden von Hirano et al auch nur K_{M} und Vₘₐₓ Werte für NADP⁺ (0,4 bzw.0.2) nicht aber für NADPH offenbart. Der Fachmann erkennt somit, dass das von Hirano et al beschriebene Enzym nicht zur Katalyse der Reduktion von DHCS in 7-Position zu 3,12-Diketo-7ß-CS geeignet ist.

Obwohl das Genom von *Colinsella aerofaciens* ATCC 25986 bereits 2007 sequenziert wurde (Uni Prof Data base, Sudarsanam, P. et al., 17. April 2007, Accession No. A4ECA9), konnte darin keines der analysierten Sequenzmotive einer potentiellen Short-Chain Dehydrogenase, zu weicher Enzymfamilie die HSDH Enzyme zählen, zugeordnet werden.

Carrea et al beschreiben in Biotechnology Letters 1992, 14,12, 1131-1135 ein Verfahren zur Herstellung von UDCS aus CS unter Verwendung eine angereicherten 7ß-HSDH und einer 3α-HSDH. Nachteilig bei diesem Verfahren ist jedoch die Verwendung einer 7ß-HSDH aus dem pathogenen Mikroorganismus *Clostridium absonum,* sowie die Notwendigkeit, das Enzym aus dem bakteriellen Extrakt, der zudem unerwünschte 7α-HSDH Aktivität besitzt, präparativ aufzureinigen.

Akao, T. et al beschreiben in J. Biochem, 102, 3, 613-619 (1987) die Reinigung und Charakterisierung der 7β-HSDH aus Ruminococcus sp.

Aufgabe der Erfindung ist daher die Bereitstellung eines neuen Verfahrens zur Herstellung von UDCS, welches oben geschilderte Nachteile vermeidet. Insbesondere sollte ein neues Enzym bereitgestellt werden, welches die stereospezifische Reduktion von DHCS in 7-Position zu 3,12-Diketo-7ß-CS katalysiert.

Eine weitere Aufgabe besteht in der Bereitstellung neuartiger 7ß-HSDH Enzyme weiche z.B. zur Verwendung bei der Herstellung von UDCS einsetzbar sind, und insbesondere die stereo- und enantioselektive Oxidation/Reduktion von Cholsäure-Derivaten in 7-Position katalysieren.

### Kurzfassung der Erfindung:

Obige Aufgaben konnten überraschenderweise gelöst werden durch die Isolierung und Charakterisierung einer neuartigen regio- und stereospezifischen 7ß-HSDH aus aeroben Bakterien der Gattung Collinsella, insbesondere des Stammes *Collinsella aerofaciens* und deren Einsatz bei der Umsetzung von Cholsäureverbindungen, insbesondere bei der Herstellung von UDCS.

Erfindungsgemäß wird insbesondere ein neuartiges Verfahren zur UDCS Herstellung bereitgestellt, welches schematisch wie folgt darstellbar ist:

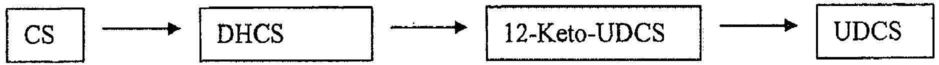

Dabei erfolgt die Oxidation von CS auf klassisch chemischem Weg in einfacher Weise. Die DHCS wird von 7β-HSDH und 3α-HSDH einzeln nacheinander oder in einem Topf zu 12-Keto-UDCS reduziert. Kombiniert mit der Wolff-Kishner-Reduktion kann UDCS somit in nur drei Schritte aus CS synthetisiert werden.

Vorteile sind unter anderem dabei, dass die enzymatische Reaktion hohen Umsatz ermöglicht, eine hohe Selektivität zeigen und keine Nebenprodukte verursachen. Die rekombinant hergestellten Enzyme 7β-HSDH und 3α-HSDH ermöglichen in überraschend vorteilhafter Weise die Massenproduktion von UDCS.

### Figurenbeschreibung:

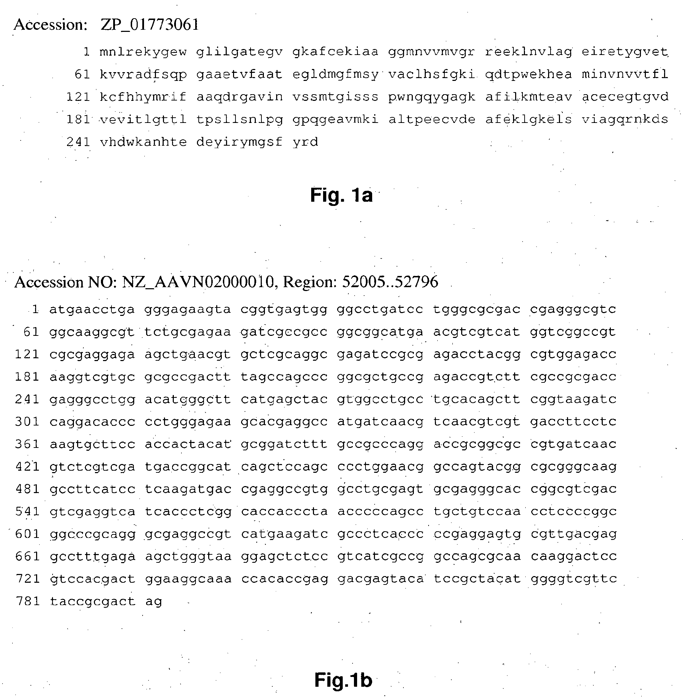

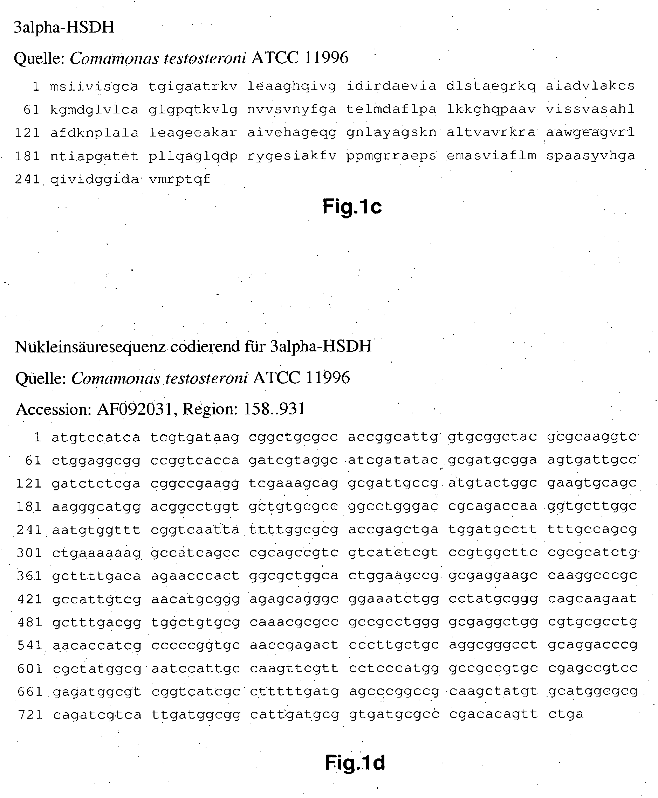

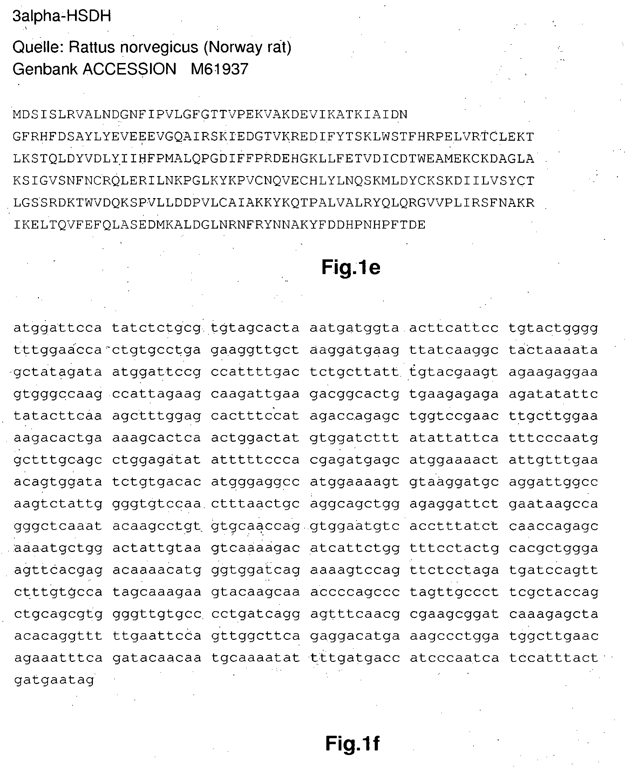

Figur 1a zeigt die Aminosäuresequenz der 7ß-HSDH aus Collinsella aerofaciens und Figur 1b die kodierende Nukleinsäuresequenz zur Aminosäuresequenz von Figur 1a; Figur 1c zeigt die Aminosäuresequenz der 3α-HSDH aus *Comanomonas testosteroni* und Figur 1d die kodierende Nukleinsäuresequenz zur Aminosäuresequenz von Figur 1c; Figur 1e zeigt die Aminosäuresequenz der 3α-HSDH aus *Rattus norvegicus* und Figur 1f die kodierende Nukleinsäuresequenz zur Aminosäuresequenz von Figur 1 e.

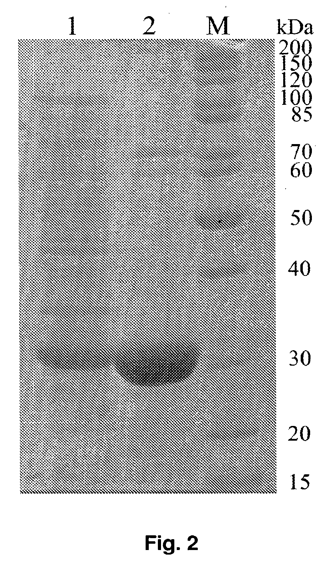

Figur 2 zeigt das SDS-Gel einer erfindungsgemäß hergestellten, gereinigten 7β-HSDH und zwar auf Bahn 1: Zellrohextrakt; Bahn 2: gereinigtes Protein; Bahn M: Page Rouler™, Molekulargewichtsmarker (Fermentas, Deutschland).

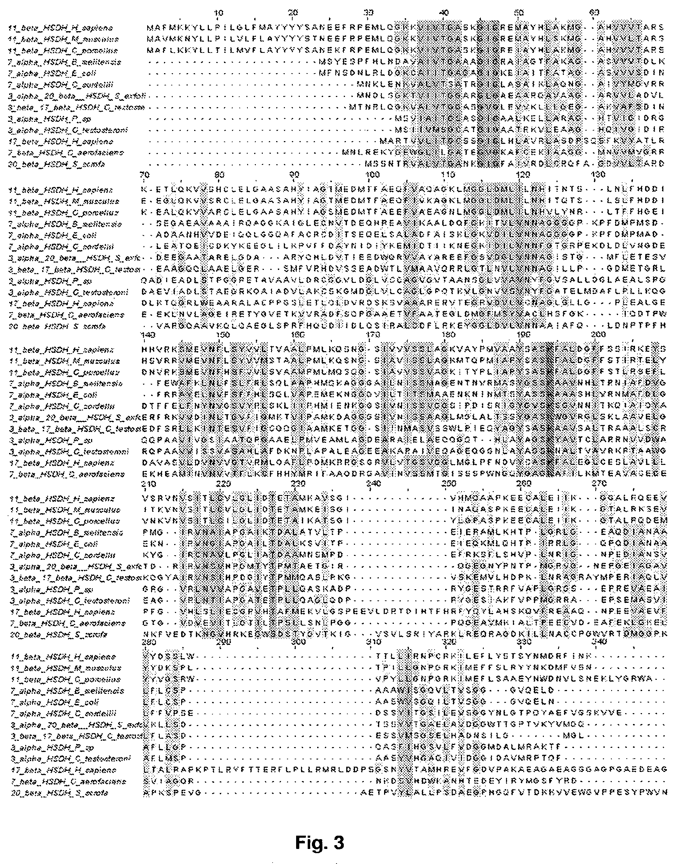

Figur 3 zeigt das Sequenzalignment von 7β-HSDH aus *Collinsella aerofaciens* DSM 3979 und ausgewählten HSDH-Proteinen. Konservierte Reste in den Sequenzen sind farblich hervorgehoben. Die folgenden Sequenz-Hinterlegungsnummern gelten: 11β-HSDH von *Homo sapiens,* GenBank NP_005516; 11β-HSDH von *Mus_musculus*, GenBank NP_001038216; 11β-HSDH von *Cavia porcellus,* GenBank AAS47491; 7α-HSDH von *Brucella melitensis,* GenBank NP_698608; 7α-HSDH von *Escherichia coli,* GenBank NP_288055; 7α-HSDH von *Clostridium sordellii,* GenBank P50200; 3α/20β-HSDH von *Streptomyces exfoliates,* Swiss-Port P19992; 3β/17β-HSDH von *Comamonas testosteroni* GenBank AAA25742; 3α-HSDH von *Pseudomonas_sp,* GenBank BAA08861; 3α-HSDH von *Comamonas festosteroni,* GenBank YP_003277364; 17β-HSDH von *Homo sapiens,* GenBank NP_000404; 20β-HSDH von *Sus scrofa,* GenBank NP_999238. Die letzten 17 Aminosäuren von 17β-HSDH aus *Homo sapiens* und die letzte Aminosäure von 20β-HSDH aus *Sus scrofa* sind in dem Alignment nicht mehr gezeigt, da daraus keine zusätzlichen Alignmentinformationen ableitbar waren.

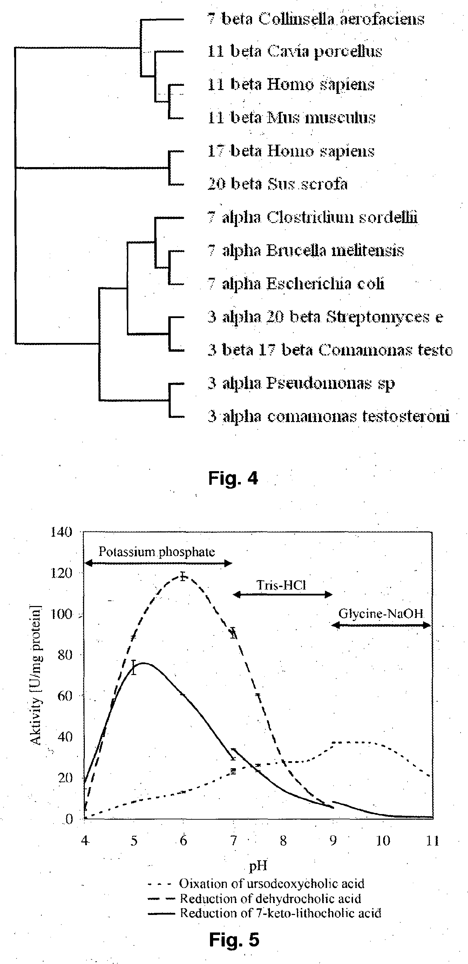

Figur 4 zeigt den phylogenetischen Stammbaum auf der Grundlage eines Alignments von HSDH-Proteinsequenzen und veranschaulicht die Verwandtschaft zwischen den ausgewählten HSDH-Proteinen.
Figur 5 veranschaulicht den Einfluss des pH-Werts auf die Aktivität der erfindungsgemäßen 7β-HSDH. Folgende Testbedingungen wurden verwendet: pH 4 bis 7 in 50 mM Kaliumphosphat; pH 7 bis 9 in 50 mM Tris-HCl; pH 9 bis 11 in 50 mM Glycin-NaOH bei 23°C. Die Aktivitätswerte für DHCS und 7-Keto-LCS bei pH 9 bis 11 in 50 mM Glycin-NaOH überlappen.

### Spezielle Ausführungsformen

1. Die Erfindung betrifft eine 7β-Hydroxysteroiddehydrogenase (7β-HSDH) mit den Merkmalen des Anspruchs 1. Diese ist insbesondere erhältlich aus einem anaeroben Bakterium, insbesondere der Gattung *Collinsella,* wie dem Stamm *Collinsella aerofaciens* DSM 3979 (ATCC 25986).
Die aus *Collinsella aerofaciens* DSM 3979 erhältliche 7β-HSDH ist zusätzlich insbesondere durch wenigstens eine weitere der folgenden Eigenschaften, wie z.B. 2, 3, 4, 5, 6 oder 7 oder aller solcher Eigenschaften, gekennzeichnet:
a) Molekulargewicht (SDS-Gelelektrophorese): etwa 28-32 kDa, insbesondere etwa 29 bis 31 kDa oder etwa 30 kDa;
b) Molekulargewicht (Gelfiltration, unter nicht denaturierenden Bedingungen, wie insbesondere ohne SDS): etwa 55 bis 57 kDa, wie 56.1 kDa, womit sich das erfindungsgemäße Enzym deutlich von dem von Hirano et al (s.o.) beschriebenen 7ß-HSDH Enzym aus C. *aerofa*ciens ATCC25986 mit 45kDa unterscheidet. Dies belegt die dimere Beschaffenheit (Quartätstruktur) der 7β-HSDH aus *Collinsella aerofaciens* DSM 3979;
c) Stereoselektiven Reduktion der 7-Carbonyl-Gruppe von 7-Keto-LCS in eine 7β-Hydroxy-Gruppe;
d) pH Optimum für die Oxidation von UDCS im Bereich von pH 8,5 bis 10,5, insbesondere 9 bis 10;
e) pH Optimum für die Reduktion von DHCS und 7-Keto-LCS im Bereich von pH 3,5 bis 6,5, insbesondere bei pH 4 bis 6, wodurch überraschend die Möglichkeit gegeben ist oxidative (Merkmals d)) und reduktive Prozesse durch Wahl des pH-Wertes zu beeinflussen;
f) wenigstens einen kinetischen Parameter aus folgender Tabelle für wenigstens eines der dort genannten Substrate/Cofaktoren; im Bereich von ±20%, insbesondere ±10%, ±5%, ±3% ±2% oder ±1% um den in der folgenden Tabelle jeweils konkret genannten Wert.

| | K_{M} (µM) | Vₘₐₓ (U/mg Protein)^{b)} | k_{cat} (1 µmol/(µmol×min)) |
|---|---|---|---|
| NADP^{+c)} | 5.32 | 30.58 | 944.95 |
| NADPH^{c)} | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| NAD⁺ | -^{a)} | - | Spuren |
| NADH | - | - | Spuren |

| | | | |
|---|---|---|---|
| ^{a)} konnten aufgrund der sehr geringen Aktivität nicht bestimmt werden ^{b)} 1 U = 1 µmol/min ^{c)} womit sich das erfindungsgemäße Enzym deutlich von dem von Hirano et al (s.o.) beschriebenen 7ß-HSDH Enzym aus C. aerofaciens ATCC25986 unterscheidet, wofür deutlich niedrigere K_{M} und Vₘₐₓ Werte (0,4 bzw 0,2) beschrieben wurde und keine Aktivität für NADPH gemessen wurde. | | | |

g) Phylogenetische Sequenzverwandtschaft der prokaryotischen 7β-HSDH aus *Collinsella aerofaciens* DSM 3979 mit der tierischen 11β-HSDH-Untergruppe, umfassend *Cavia porcellus*, *Homo sapiens* und *Mus musulus*, verwandt.
Beispielsweise zeigt eine 7β-HSDH folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f).
2. 7ß-HSDH oder davon abgeleitetes funktionales Äquivalent nach Ausführungsform 1, welche(s) anders als das von Hirano et al (s.o.) beschriebenen 7ß-HSDH Enzym aus C. aerofaciens ATCC25986
a) die stereospezifische Reduktion (Hydrierung) eines 7-Ketosteroids zum korrespondierenden 7ß-Hydroxysteroid, und
b) die regiospezifische Hydrierung (Reduktion) eines Ketosteroids umfassend eine Ketogruppe in 7-Position und wenigstens eine weitere Ketogruppe am Steroidgerüst zum korrespondierenden 7ß-Hydroxysteroid, wie insbesondere von Dehydrocholsäure (DHCS) in 7-Position zur korrespondierenden 3,12-Diketo-7ß-Cholansäure, katalysiert, und z.B. NADPH abhängig ist, ist auch hierin beschrieben.

3. Zudem beschriebenist eine 7ß-HSDH mit einer Aminosäuresequenz gemäß SEQ ID NO:2 (Accession NO: ZP_01773061) oder einer davon abgeleiteten Sequenz mit einem Identitätsgrad von wenigstens 80%, wie z.B. wenigstens 85, oder 90, wie z.B. wenigstens 91, 92, 93, 94, 95, 96, 97, 98, 99 oder 99,5% zu dieser Sequenz; gegebenenfalls zusätzlich gekennzeichnet durch eine der folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f) gemäß obiger Definition.
4. Hierin beschrieben ist auch eine Nukleinsäuresequenz kodierend für eine 7ß-HSDH gemäß der Definition nach einer der vorhergehenden Ausführungsformen, wie z.B. gemäß SEQ ID NO:1 (Accession NO: NZ_AAVN02000010, Region: 52005..52796)
5. Hierin beschrieben ist auch eine Expressionskassette, umfassend eine Nukleinsäuresequenz gemäß Ausführungsform 4 unter der genetischen Kontrolle wenigstens einer regulativen Nukleinsäuresequenz.
6. Hierin beschrieben ist auch ein Vektor, umfassend wenigstens eine Expressionskassette nach Ausführungsform 5.
7. Hierin beschrieben ist auch ein rekombinanter Mikroorganismus, der wenigstens eine Nukleinsäuresequenz gemäß Ausführungsform 4 oder wenigstens eine Expressionskassette nach Ausführungsform 5 trägt oder mit wenigstens einem Vektor nach Ausführungsform 6 transformiert ist.
8. Beschrieben ist auch ein Verfahren zur Herstellung einer 7ß-HSDH nach einer der Ausführungsformen 1 bis 3, wobei man einen Mikroorganismus nach Ausführungsform 7 kultiviert und die exprimierte 7ß-HSDH aus der Kultur isoliert.
9. Die Erfindung betrifft auch ein Verfahren zur enzymatischen Synthese von 7β-Hydroxysteroiden, wobei man das korrespondierende 7-Ketosteroid in Gegenwart einer 7β-HSDH gemäß der Definition der Ausführungsform 1 umsetzt, und wenigstens ein gebildetes Reduktionsprodukt aus dem Reaktionsansatz gegebenenfalls isoliert.
10. Die Erfindung betrifft auch ein Verfahren nach Ausführungsform 9, wobei das zu reduzierende Ketosteroid ausgewählt ist unter
Dehydrocholsäure (DHCS),
7-Keto-lithocholsäure (7-Keto-LCS),
7,12-Diketo-lithocholsäure (7,12-Diketo-LCS) und
den Derivaten davon, wie insbesondere einem Salz, Amid oder Alkylester der Säure.
11. Die Erfindung betrifft auch ein Verfahren nach Ausführungsform 10, wobei DHCS oder ein Derivat davon zu 3,12-Diketo-7ß-cholansäure oder zum entsprechenden Derivat umgesetzt wird; oder
7-Keto-LCS oder ein Derivat davon zu Ursodeoxycholsäure (UDCS) oder zum entsprechenden Derivat umgesetzt wird; oder
7,12-Diketo-LCS oder ein Derivat davon zu 12-Keto-ursodeoxycholsäure (12-Keto-UDCS) umgesetzt wird.
12. Die Erfindung betrifft auch ein Verfahren nach einer der Ausführungsformen 9 bis 11, wobei die Reduktion in Gegenwart (und unter Verbrauch) von NAD(P)H erfolgt.
13. Die Erfindung betrifft auch ein Verfahren zur enzymatischen Oxidation von 7β-Hydroxysteroiden, wobei man das Hydroxysteroid in Gegenwart einer 7β-Hydroxysteroiddehydrogenase gemäß der Definition der Ausführungsform 1 umsetzt, und ein gebildetes Oxidationsprodukt aus dem Reaktionsansatz gegebenenfalls isoliert.
14. Die Erfindung betrifft auch ein Verfahren nach Ausführungsform 13, wobei das 7β-Hydroxysteroid 3,12-Diketo-7ß-CS oder ein Derivat davon, wie insbesondere ein Salz, Amid oder Alkylester, ist.
15. Die Erfindung betrifft auch ein Verfahren nach einer der Ausführungsformen 13 und 14, wobei die Oxidation in Gegenwart (und unter Verbrauch) von NAD(P)⁺ erfolgt.
16. Die Erfindung betrifft auch ein Verfahren nach einer der Ausführungsformen 12 und 15, wobei die verbrauchten Redox-Äquivalente elektrochemisch oder enzymatisch, insbesondere in situ, regeneriert werden.
17. Die Erfindung betrifft auch ein Verfahren nach Ausführungsform 16, wobei verbrauchtes NAD(P)H durch Kopplung mit einem NAD(P)H-regenerierenden Enzym ausgewählt unter einer NAD(P)H Dehydrogenase und insbesondere einer Alkoholdehydrogenase (ADH), insbesondere in situ, regeneriert wird.
18. Die Erfindung betrifft auch ein Verfahren nach Ausführungsform 17, wobei das NAD(P)H-regenerierende Enzym ausgewählt ist unter natürlichen oder rekombinanten, isolierten oder angereicherten
a) Alkoholdehydrogenasen (ADH; EC.1.1.1.2) und
b) davon abgeleiteten funktionalen Äquivalenten (insbesondere funktionalen Domänen).

19. Die Erfindung betrifft auch ein Verfahren zur Herstellung von Ursodesoxycholsäure (UDCS) der Formel (1) 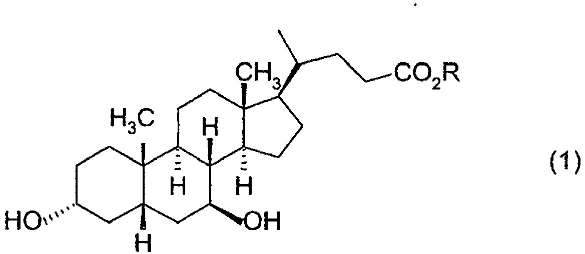 worin
R für Alkyl, NR¹R², H, ein Alkalimetallion oder N(R³)₄⁺steht, worin die Reste R³ gleich oder verschieden sind und für H oder Alkyl stehen,
wobei man
a) gegebenenfalls eine Cholsäure (CS) der Formel (2) 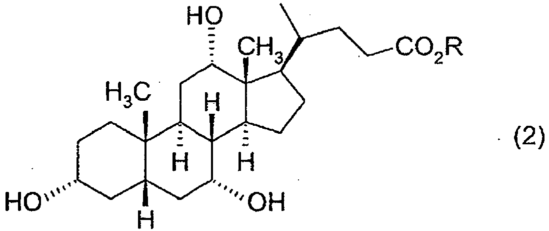 worin R die oben angegebenen Bedeutungen besitzt, zur Dehydrocholsäure (DHCS) der Formel (3) 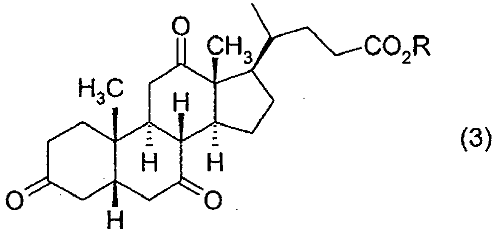 worin R die oben angegebenen Bedeutungen besitzt, chemisch oxidiert;
b) DHCS in Gegenwart wenigstens einer 7ß-HSDH gemäß der Definition der Ausführungsform 1 und 2 NADPH-abhängig zur 3,12-Diketo-7ß-cholansäure (3,12-Diketo-7ß-CS) der Formel (4) 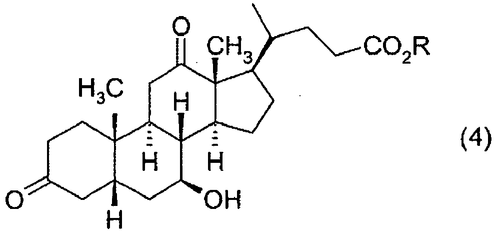 reduziert,
c) 3,12-Diketo-7ß-CS in Gegenwart wenigstens einer 3α-Hydroxysteroiddehydrogenase (3α-HSDH) NADPH-abhängig oder NADH-abhängig (je nach Typ der verwendeten 3α-HSDH) zur korrespondierenden 12-Keto-Ursodesoxychiolsäure (12-Keto UDCS) der Formel (5) 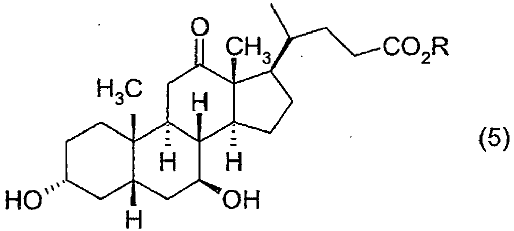 worin R die oben angegebenen Bedeutungen besitzt, reduziert und anschließend
d) 12-Keto-UDCS der Formel (5) chemisch zu UDCS reduziert; und
e) das Reaktionsprodukt gegebenenfalls weiter aufreinigt.

20. Die Erfindung betrifft auch ein Verfahren nach Ausführungsform 19, wobei die Schritte b) und/oder c) in Gegenwart von Reduktionsäquivalenten (NADPH und/oder NADH) durchgeführt werden.
21. Die Erfindung betrifft auch ein Verfahren nach einer der Ausführungsformen 19 und 20, wobei die Schritte b) und/oder c) mit einem (insbesondere enzymatischen) Cofaktorregenerierungsschritt gekoppelt sind.
22. Die Erfindung betrifft auch ein Verfahren nach Ausführungsform 21, wobei Schritt b) mit einem Cofaktorregenerierungsschritt, insbesondere in situ, gekoppelt ist, bei welchem NADPH durch Alkoholdehydrogenase (ADH) unter Verbrauch eines Opferalkohols (insbesondere Isopropanol und Bildung von Aceton) regeneriert wird, und wobei man gegebenenfalls die Entfernung von Aceton aus dem Reaktionsgleichgewicht (z.B. durch Temperaturerhöhung) begünstigt.
23. Die Erfindung betrifft auch ein Verfahren nach Ausführungsform 21 oder 22, wobei Schritt c) mit einem Cofaktorregenerierungsschritt, insbesondere in situ, gekoppelt ist, bei welchem je nach Typ der verwendeten 3α-HSDH NADH durch Formiatdehydrogenase (FDH) unter Verbrauch von Formiat (und Bildung von gasförmigem CO₂) regeneriert wird; oder bei welchem NADPH durch Alkoholdehydrogenase (ADH) unter Verbrauch eines Opferalkohols (insbesondere Isopropanol und Bildung von Aceton) regeneriert wird und wobei man gegebenenfalls die Entfernung von Aceton aus dem Reaktionsgleichgewicht (z.B. durch Temperaturerhöhung) begünstigt
24. Die Erfindung betrifft auch ein Verfahren nach einer der Ausführungsformen 8 bis 23, wobei die Reaktion mit wenigsetns einem der beteiligten Enzyme in immobilisierter Form durchgeführt wird.
25. Hierin beschrieben ist auch ein Bioreaktor, umfassend eine 7β-Hydroxysteroiddehydrogenase nach einer der Ausführungsformen 1 bis 3 in immobilisierter Form.

### Weitere Ausgestaltungen der Erfindung

### 1. Allgemeine Definitionen und verwendete Abkürzungen

In folgender Tabelle sind die Strukturformeln, deren chemischen Namen und die verwendeten Abkürzungen tabellarisch zusammengefasst:

| Formel | Abkürzung | Chemischer Name |
|---|---|---|
| 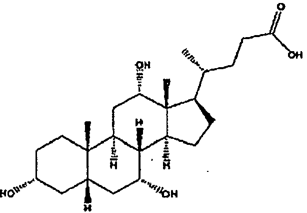 | CS | Cholsäure |
| Cholsäure | | |
| 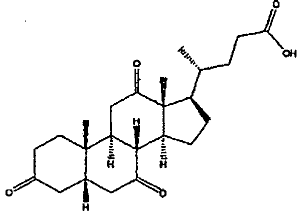 | DHCS | Dehydrocholsäure |
| Dehydrocholsäure | | |
| 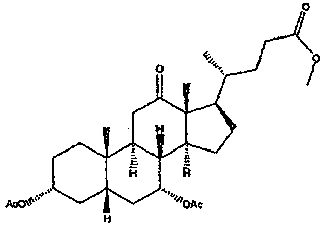 | 12-Keto-3,7-Diacetyl-CS-methylester | 12-Keto-3,7-Diacetyl-Cholansäure-methylester* |
| 12-Keto-3,7-Diacetyl-Cholansäure-methylester | | |
| 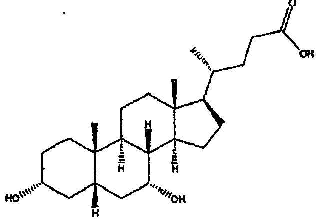 | CDCS | Chenodeoxycholsäure |
| Chenodeoxycholsäure | | |
| 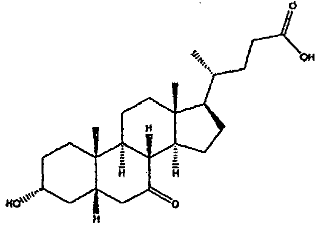 | 7-Keto-LCS | 7-Keto-Lithocholsäure |
| 7-Keto-Lithocholsäure | | |
| 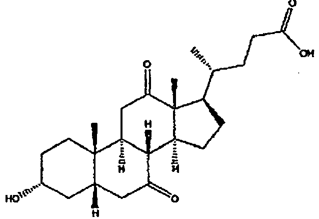 | 7,12-Diketo-LCS | 7,12-Diketo-Lithocholsäure |
| 7,12-Diketo-Lithocholsäure | | |
| 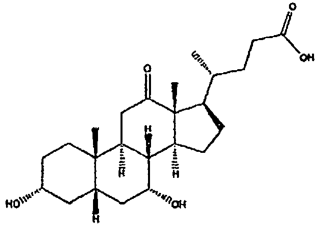 | 12-Keto-CDCS | 12-Keto-Chenodeoxycholsäure |
| 12-Keto-Chenodeoxycholsäure | | |
| 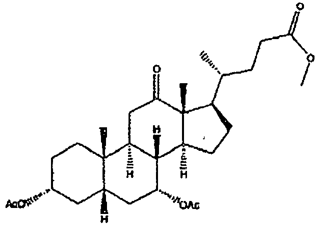 | 12-Keto-3,7-Diacetyl-CS-methylester | 12-Keto-3,7-Diacetyl-Cholansäure-methylester* |
| 12-Keto-3,7-Diacetyl-Cholansäure-methylester | | |
| 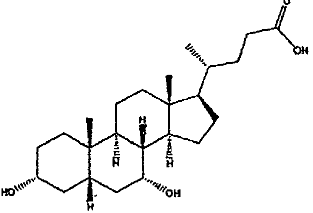 | CDCS | Chenodeoxycholsäure |
| Chenodeoxycholsäure | | |
| 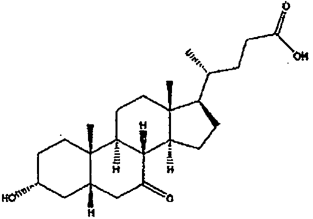 | 7-Keto-LCS | 7-Keto-Lithocholsäure |
| 7-Keto-Lithocholsäure | | |
| 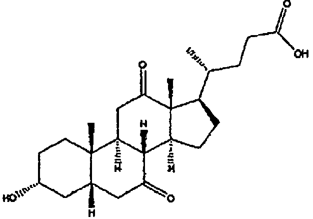 | 7,12-Diketo-LCS | 7,12-Diketo-Lithocholsäure |
| 7,12-Diketo-Lithocholsäure | | |
| 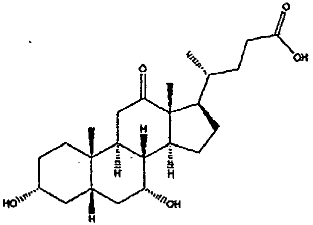 | 12-Keto-CDCS | 12-Keto-Chenodeoxycholsäure |
| 12-Keto-Chenodeoxycholsäure | | |

Werden keine anderen Angaben gemacht, so bezeichnet der Begriff "7ß-HSDH" ein Dehydrogenaseenzym, welches wenigstens die stereospezifische und/oder regiospezifische Re-duktion von DHCS zu 3,12-Diketo-7ß-CS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. Characterization of NADP-Dependent 7 ß-Hydroxysteroid Dehydrogenases from Peptostreptococcus productus and Eubacterium aerofaciens. S Hirano and N Masuda. Appl Environ Microbiol. 1982; wobei jedoch dort keine 7ß-HSDH aus *Eubacterium aerofaciens* nachgewiesen werden konnte, welche die Reduktion von 7-Ketogruppen ermöglicht). Enzyme dieser Aktivität sind unter der EC Nummer 1.1.1.201 klassifiziert.

Werden keine anderen Angaben gemacht, so bezeichnet der Begriff "3α-HSDH" ein Dehydrogenaseenzym, welches wenigstens die stereospezifische und/oder regiospezifische Reduktion von 13,2-Diketo-7ß-CS zu 12-Keto-UDCS, insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus Comanomonas testosteroni (z.B. ATCC11996) erhältlich. Eine NADPH abhängige 3α-HSDH ist z.B. aus der Nagem bekannt und ebenfalls einsetzbar. (Cloning and sequencing of the cDNA for rat liver 3 alpha-hydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie z.B. der Biuret-Methode oder dem Proteinnachweis nach Lowry et al.(vgl. Beschreibung in R.K. Scopes, Protein Purification, Springer Verlag, New York, Heidelberg, Berlin (1982)).

Unter einem "Redoxäquivalent" versteht man eine als Elektronendonor bzw. Elektronenakzeptor brauchbare, niedermolekulare organische Verbindung, wie beispielsweise Nikotinamidderivate wie NAD⁺ und NADH⁺ bzw. deren reduzierte Formen NADH bzw. NADPH. NAD(P)⁺ steht dabei für NAD⁺ und/oder NADP⁺ und NAD(P)H steht dabei für NADH und/oder NADPH.

Unter einer "Cholsäure-Verbindung" versteht man erfindungsgemäß Verbindungen mit dem Kohlenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das NH₄⁺-Salz bzw. solche Ammoniumsalze umfasst, worin wenigstens ein Wasserstoffatom durch einen C₁-C₆-Alkylrest ersetzt sein kann. Typische Alkylreste sind insbesondere C₁-C₄-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" erfindungsgemäßer Verbindungen sind insbesondere Niedrigalkyl-Ester, wie z.B. C₁-C₆-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäßer Säuren mit Ammoniak oder primären oder sekundären Monoaminen. Derartige Amine sind beispielsweise Mono- oder Di-C₁-C₆-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

Erfindungsgemäße "Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkemigen aromatischem Ring, wobei geeignete Substituenten z.B. ausgewählt sind unter Hydroxy-, Halogen (wie F, Cl, Br, J)-, Nitro- und C₁-C₆-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

Unter einer "Immobilisierung" versteht man erfindungsgemäß die kovalente oder nichtkovalente Bindung eines erfindungsgemäß verwendeten Biokatalysators, wie z.B. einer 7β-HSDH an einem festen, d.h. in dem umgebenden flüssigen Medium im Wesentlichen unlöslichen, Trägermaterial.

### 2. Proteine

Die vorliegende Erfindung ist nicht auf die konkret offenbarten Proteine bzw. Enzyme mit 7β-HSDH-Aktivität oder 3α-HSDH_Aktivität beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 7ß-HSDH-Aktivität, besitzen.

So versteht man beispielsweise unter "funktionalen Äquivalenten" Enzyme, die im verwendeten Test auf 7ß-HSDH-Aktivität eine um mindestens 1% , wie z.B. mindestens 10% oder 20 %, wie z.B. mindestens 50 % oder 75% oder 90 % höhere oder niedrigere Aktivität eines Enzyms, umfassend eine hierin definierte Aminosäuresequenz aufweisen. Funktionale Äquivalente sind außerdem vorzugsweise zwischen pH 4 bis 11 stabil und besitzen vorteilhaft ein pH-Optimum in einem Bereich von pH 4 bis 6 oder pH 6 bis 10, wie z.B . 8,5 bis 9,5, sowie ein Temperaturoptimum im Bereich von 15°C bis 80°C oder 15°C bis 40°C, 20°C bis 30°C oder 20°C bis 70°C, wie z.B. etwa 45 bis 60°C oder etwa 50 bis 55°C.

Die 7ß-HSDH -Aktivität kann mit Hilfe verschiedener bekannter Tests nachgewiesen werden. Ohne darauf beschränkt zu sein, sei ein Test unter Verwendung eines Referenzsubstrates, wie z. B. CS oder DHCS, unter standardisierten Bedingungen wie im experimentellen Teil definiert, zu nennen.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere, wie z.B. 1, 2, 3, 4, 5, 6, 7, 8, 9, 10, 11, 12, 13, 14 oder 15, Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Proteine können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Proteine welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Proteine, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäße "funktionale Äquivalente" sind Homologe zu den konkret offenbarten Proteinen. Diese besitzen insbesondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:9056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3. Nukleinsäuren und Konstrukte

### 3.1 Nukleinsäuren

Hierin beschrieben sind auch Nukleinsäuresequenzen, die für ein Enzym mit 7ß-HSDH oder 3α-HSDH-Aktivität kodieren.

Beschrieben werden auch Nukleinsäuren mit einem bestimmten Idetitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

### Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

### Pairwise alignment parameter:

| | |
|---|---|
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Koike, Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Hierin beschrieben sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Es werden sowohl isolierte Nukleinsäuremoleküle, welche für erfindungsgemäße Enzyme oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäß kodierenden Nukleinsäuren verwendet werden können hierin beschrieben.

Die erfindungsgemäß eingesetzten Nukleinsäuremoleküle können zudem untranslatierte Sequenzen vom 3'- und/oder 5'-Ende des kodierenden Genbereichs enthalten

Weiterhin beschrieben werden die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer hierin beschriebenen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden. Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den hierin beschriebenen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der hierin beschriebenen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Harnes and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH 7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Weiterhin beschrieben sind Derivate der konkret offenbarten oder ableitbaren Nukleinsäuresequenzen.

So können weitere Nukleinsäuresequenzen z.B. von SEQ ID NO:1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Hierin beschrieben sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Beschrieben werden auch durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Beschrieben werden auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten hierin beschriebener Nukleinsäuresequenzen mit der Sequenz SEQ ID NO: 1 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der hierin beschriebenen Nukleinsäuresequenzen, insbesondere der SEQ ID NO: 1, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO: 1 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 1 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise
- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

### 3.2 Konstrukte

Hierin beschrieben sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Unter einer "Expressionseinheit" wird hierin eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer regulative Nukleinsäuresequenz". Zusätzlich zum Promotor können weitere, regulative Elemente, wie z.B. Enhancer, enthalten sein.

Unter einer "Expressionskassette" oder "Expressionskonstrukt" wird hierin eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäß verwendeten Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionelle" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäß verwendete Nukleinsäurekonstrukte umfassen insbesondere Sequenz SEQ ID NO: 1 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäß verwendeten Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, lacl^{q-,} T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFal-pha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pET28a(+), pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

In einer weiteren Ausgestaltungsform des Vektors kann der das erfindungsgemäß verwendete Nukleinsäurekonstrukt oder die erfindungsgemäß verwendete Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäß verwendeten Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäß verwendeten Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 4. Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der hierin beschriebenen Vektoren sind rekombinante Mikroorganismen herstellbar, welche beispielsweise mit wenigstens einem hierin beschriebenen Vektor transformiert sind und zur Produktion der erfindungsgemäßen Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäß verwendete Nukleinsäure oder das Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit 7ß-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden. 5. Herstellung der UDCS

### 1. Schritt: Chemische Umsetzung von CS zu DHCS

Die Hydroxygruppen von CS werden mit Chromsäure bzw. Chromaten in saurer Lösung (z.B. H₂SO₄) zu Carbonylgruppe in an sich bekannter Weise auf klassisch-chemischem Weg oxidiert. Dadurch entsteht DHCS.

### 2. Schritt: Enzymatische Umsetzung von DHCS zu 12-Keto-UDCS

In wässriger Lösung wird DHCS durch 3α-HSDH und 7β-HSDH spezifisch zu 12-Keto-UDCS in Anwesenheit von NADPH bzw. NADH reduziert. Der Cofaktor NADPH bzw. NADH kann durch eine ADH bzw. FDH von Isopropanol bzw. Natium-formiat regeneriert werden. Die Reaktion läuft unter milder Bedingung. Beispielseweise kann die Reaktion bei pH = 4 bis 9, 6 bis 9 oder 7 bis 9, insbesondere etwa pH = 8 und bei etwa 10 bis 30, 25 bis 25 oder etwa 23°C durchgeführt werden.

### 3. Schritt Chemische Umsetzung von 12-Keto-UDCS zu UDCS

Die 12-Carbonylgruppe von 12-Keto-UDCS wird mittels Wolff-Kishner-Reduktion in an sich bekannter Weise entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200 °C erhitzt, hierbei wird Stickstoff abgespaltet und UDCS entsteht.

### 6. Rekombinante Herstellung der HSDH

Hierin beschrieben sind weiterhin Verfahren zur rekombinanten Herstellung erfindungsgemäßer Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezemiert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

### Experimenteller Teil:

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wie bei Sambrook et al. (1989) a.a.O. beschrieben durchgeführt werden.

### 1. Allgemeine Angaben

### Materialien:

Die genomische DNA von *Collinsella aerofaciens* DSM 3979 (ATCC 25986, frühere Bezeichnung *Eubacterium aerofaciens*) wurde von der Deutschen Sammlung für Mikroorganismen und Zellkulturen (DSMZ) bezogen. UDCS und 7-Keto-LCS sind an sich bekannte und in der Literatur beschriebene Ausgangsverbindungen. Alle übrigen Chemikalien wurden von Sigma-Aldrich und Fluka (Deutschland) bezogen. Sämtliche Restriktionsendonukleasen, die T4 DNA Ligase, die *Taq* DNA Polymerase und Isopropyl-β-D-1-thiogalactopyranosid (IPTG) wurden von Fermentas (Deutschland) bezogen.

### Medien:

LB-Medium, enthaltend Trypton 10 g, Hefeextrakt 5 g, NaCl 5 g pro Liter Medium

### Expressionsvektoren

pET22b(+) bzw. pET28a(+) (Novagen, Madison, Wisconsin, USA).

### Mikroorganismen

Der Escherichia. coli Stamm DH5α (Novagen, Madison, Wisconsin, USA) wurde bei 37°C in LB-Medium, enthaltend geeignete Antibiotika vermehrt.

Der *Escherichia coli* Stamm BL21(DE3) (Novagen, Madison, Wisconsin, USA) wurde bei 37°C in LB-Medium, enthaltend geeignete Antibiotika, vermehrt und wurde nach Induktion bei OD₆₀₀ = 0.8 mit 0.5 mM IPTG bei 25°C und 140 Upm gehalten.

### Analytische Methoden

### 1. Standardbedingungen für 7ß-HSDH Aktivitätsbestimmung

Die Reaktionsmischung enthält ein Gesamtvolumen von 1 ml:
- 880 µl: 50 mM Kaliumphosphat Puffer, pH 8.0
- 10 µl: 10 mM UDCS (gelöst in Wasser, pH 8)
- 10 µl: Enzymlösung (in Puffer wie oben, im Bereich von 1 zu 10 U/ml)
- 100 µl: 1 mM NADP+ (in Puffer wie oben)
Die Zunahme der Extinktion bei 340 nm wird gemessen und die Aktivität wird als Enzym-Unit (U, i.e. µmol/min) unter Verwendung des molaren Extinktionskoeffizienten von 6.22 mM⁻¹×cm⁻¹, berechnet,

### 2. Proteinbestimmung mittels BCA-Assay

Die Proben wurden mit BCA-Reagenz (von Interchim) gemischt und bei 37°C für 45 min inkubiert. Der Proteingehalt wurde bei 562 nm gegen einen Kalibrationskurve (BSA) im Konzentrationsbereich des verwendeten Assay bestimmt.

### 3. Dünnschichtchromatographie

5 bis 10 µg Probe wurden auf eine DC-Folie Kieselgel 60 (Merck) aufgetragen. Authentische Substanzen wurden als Referenz aufgetragen. Ein Ende der DC-Folie wurde in Laufmittel eingetaucht bis die Mobilphase oben erreicht. Die DC-Folie wurde getrocknet und mittels Molybdatophosphorsäure entwickelt.

### 2. Beispiele

### Herstellungsbeispiel 1: Identifizierung der 7β-HSDH Aktivität

Die genomische DNA-Sequenz von *Collinsella aerofaciens* ATCC 25986 wurde im Jahr 2007 von "Washington University Genome Sequencing Center" für das "human gut microbiome project" bei GenBank veröffentlicht. HSDHs gehören zu den "Short-Chain Dehydrogenasen". Da die biochemische Funktion der "Short-Chain Dehydrogenase" aus *Collinsella aerofaciens* ATCC 25986 nicht in GenBank annotiert wurde, wurden 9 Kandidaten in Vektor pET22b⁺ einkloniert, und anschließend in *E. coli* BL21(DE3) exprimiert.

Dazu wurden 7ß-HSDH kodierende Sequenzen PCR-amplifiziert. Die PCR-Produkte erhielt man unter Verwendung der genomischen DNA von *Collinsella aerofaciens* ATCC 25986 (DSM 3979) als Template und den Primer 5'-gggaattcCATATGAACCTGAGGGAGAAGTA-3' (SEQ ID NO:3) und 5'-cccAAGCTTCTAGTCGCGGTAGAACGA-3' (SEQ ID NO:4). Die *Ndel* und *Hin*dIII Schnittstellen in den Primer-Sequenzen sind unterstrichen. Das PCR-Produkt wurde mittels PCR-Purification-Kit (Qiagen) aufgereinigt und dann mit den Enzymen *Nde*I und *Hin*dIII geschnitten. Der entsprechende Vektor wurde auch mit den *Nde*I und *Hin*dIII geschnitten. Die Produkte wurden auf ein Agarose-Gel aufgetragen, aufgetrennt, aus diesem ausgeschnitten und aufgereinigt. Das geschnittene PCR-Produkt und der geschnittene Vektor wurden mittels T4-Ligase ligiert. Das Ligant wurde in E. *coli* DH5α transformiert. Der entstehende Vektor (enthält das Gen von 7ß-HSDH) würde durch Sequenzierung bestätigt und in *E. coli* BL21(DE3) transformiert und mit IPTG induziert und exprimiert.

Die Expression wurde in 50 ml LB-Medium durchgeführt. Zur Vorbereitung von Vorkultur wurde eine Kolonie auf LB-Agar-Platte in 5 ml LB-Medium (enthält entsprechende Antibiotika) gepickt und bei 37°C und 160 Upm über Nacht inkubiert. Das 50 ml LB-Medium (enthält entsprechende Antibiotika) wurde mit 500 µl Vorkultur angeimpft. Die Kultur wurde bei 37°C und 160 Upm inkubiert. Bis OD600 ca. 0,8 wurde die Expression durch Zugabe von 0,5 mM IPTG induziert. Nach 6 h oder über Nacht wurden die Zellen abzentrifugiert. Die Pellets wurden in 6 ml Kaliumphosphat-Puffer (50 mM, pH 8, enthält 0,1 mM PMSF) resuspendiert und mittels Ultraschall aufgeschlossen. Die Zelltrümmer wurden durch Zentrifugation entfernt.

Zur Identifizierung 7β-HSDH-Aktivität wurde die Aktivität mittels photometrischer Methode untersucht. In einer 1 ml Küvette wurde hierzu Enzym und 0,1 mM Testsubstanz (UDCS) in Kaliumphosphat Puffer (50 mM, pH 8) gemischt Nach Zugabe von NADPH(NADH) bzw. NADP⁺(NAD⁺) wurde Abbau oder Bildung von NAD(P)H gemessen. Ein Enzym von den 9 Kandidaten zeigt Aktivität (60 U/ml) gegen UDCS in Anwesenheit von NADP⁺, aber keine Aktivität gegen CS. Die NADPH-abhängige 7β-HSDH-Aktivität von diesem Enzym wurde identifiziert.

In der photometrischen Untersuchung zeigte die 7β-HSDH die Aktivität 60 U/ml gegen UDCS, Aktivität 35 U/ml gegen 7-Keto-LCS und Aktivität 119 U/ml gegen DHCS in Anwesenheit von NADP⁺ bzw. NADPH. Die Aktivität gegen CS ist nicht nachweisbar.

Das Gen, das die 7β-HSDH codiert, wurde in pET28a+ mit His-Tag umkloniert, um eine schnelle Reinigung zu ermöglichen. Diese 7β-HSDH mit His-Tag wurde aktiv in *E. coli* BL21(DE3) exprimiert wie oben beschrieben wurde. Die Aufreinigung wurde mit einer Talon-Säule durchgeführt. Die Säule wurde zuerst mit Kaliumphosphat-Puffer (50 mM, pH 8, mit 300 mM NaCl) equilibriert. Nachdem das Zelllysat geladen worden ist, wurde die Säule mit Kaliumphosphat-Puffer (50 mM, pH 8, mit 300 mM NaCl) gewaschen. Die 7β-HSDH wurde mit Kaliumphosphat-Puffer (50 mM, pH 8, mit 300 mM NaCl und 200 mM Imidazol) eluiert. Das Imidazol im Eluat wurde mitttels Dialyse entfernt. Die Ausbeute der Aufreinigung betrug 76% mit Reinheit ca. 90%.

### Umsetzungsbeispiel 1: Enzymatische Umsetzung von 7-Keto-LCS durch die 7β-HSDH

Zur Überprüfung der biochemischen Funktion der 7β-HSDH, wurde eine Umsetzung von 7-Keto-LCS durch die 7β-HSDH durchgeführt. Die 20 ml Umsetzung enthält 50 mM 7-Keto-LCS (ca. 0,4 g), 5 U/ml 7β-HSDH und 0,05 mM NADP⁺. Zur Regenerierung von NADPH wurde 4 U/ml ADH und 1 % Isopropanol eingesetzt (siehe Schema 1). Die Reaktion wurde bei pH 8 und 24°C unter Rühren im Abzug durchgeführt. Da Aceton schneller als Isopropanol verdunstet, wird die Reaktion in Bildung von UDCS geschoben. 1% Isopropanol wurde nach 24 h, 48 h und 72 h nachgegeben. Das Produkt wurde mittels DC analysiert (Kieselgel 60, Merck, Laufmittel Petrolether und Ethylacetat 1:10, vol:vol). Auf DC wurde das Produkt mit authentischen Referenzen 7-Keto-LCS, UDCS und CDCS verglichen. Die DC-Analyse zeigt, dass UDCS aus 7-Keto-LCS von der 7β-HSDH gebildet wurde. Das Enantiomer CDCS ist auf DC nicht nachweisbar.

Schema 1: Schematische Darstellung von Reduktion von 7-Keto-LCS durch die 7β-HSDH. Die ADH regeneriert den Cofaktor NADPH.

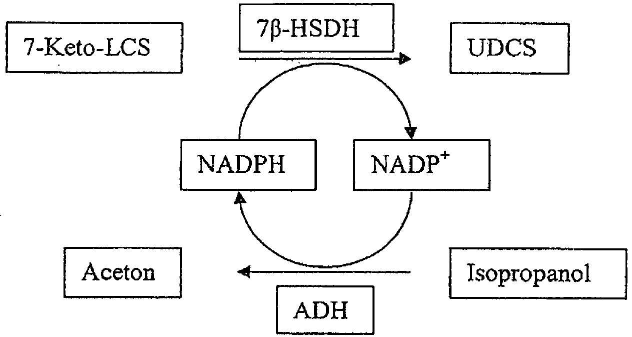

### Umsetzungsbeispiel 2: Enzymatische Herstellung von 12-Keto-UDCS aus DHCS durch 7β-HSDH

Zur Überprüfung der Einsetzbarkeit der 7β-HSDH zu Herstellung von 12-Keto-UDCS aus DHCS, wurde eine Umsetzung von DHCS durch die 7β-HSDH durchgeführt. Die 50 ml Umsetzung enthält 50 mM DHCS (1 g), 5 U/ml 7β-HSDH und 0,05 mM NADP⁺. Zur Regenerierung von NADPH wurde 4 U/ml ADH und 1% Isopropanol eingesetzt (siehe Schema 2). Die Reaktion wurde bei pH 8 und 24°C unter Rühren im Abzug durchgeführt. Da Aceton schneller als Isopropanol verdunstet, wird die Reaktion in Bildung von 3,12-Diketo-7β-CS geschoben. Um vollständige Umsetzung zu erreichen, wurde 1% Isopropanol nach 24 h, 48 h und 72 h nachgegeben. Das Zwischenprodukt 3,12-Diketo-7β-CS wurde mittels DC analysiert. Das Edukt DHCS war nicht mehr nachweisbar auf DC (Kieselgel 60, Merck; Laufmittel Chlorform:Methanol:Essigsäure 10:1:0,08 vol:vol:vol).

Schema 2: Schematische Darstellung von Reduktion von DHCS durch die 7β-HSDH. Die ADH regeneriert den Cofaktor NADPH.

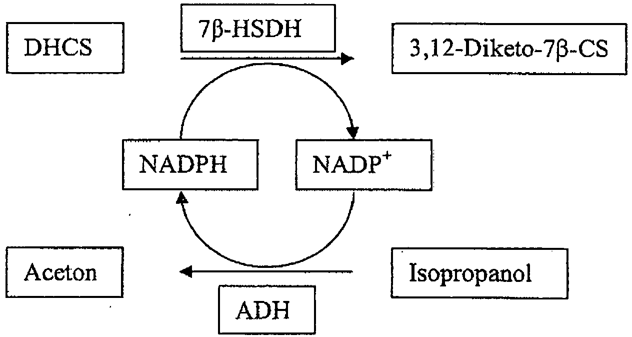

### Umsetzungsbeispiel 3: Enzymatische Umsetzung von 3,12-Diketo-7β-CS zu 12-Keto-UDCS

Das Zwischenprodukt 3,12-Diketo-7β-CS (hergestellt gemäß Umsetzungsbeispiel 2) wurde von einer 3α-HSDH (SEQ ID NO:5 und 6) aus *Comamonas testosteroni* (Mobus, E. and E. Maser, Molecular cloning, overexpression, and characterization of steroid-inducible 3alpha-hydroxysteroid dehydrogenaselcarbonyl reductase from Comamonas testosteroni. A novel member of the short-chain dehydrogenase/reductase superfamily. J Biol Chem, 1998. 273(47): p. 30888-96) weiter zu 12-Keto-UDCS umgesetzt. Diese 3α-HSDH benötigt Cofaktor NADH, der von der FDH regeneriert wurde (siehe Abb. 3). Zu der Reaktion wurden 4U/ml 3α-HSDH, 1 U/ml FDH (NADH-abhängig, Codexis), 200 mM Natriumformiat und 0,05 mM NAD⁺ zugegeben. Nach 40 h wurde das Produkt mit 2 M HCl bis pH 2 angesäuert und mit 6 mal 10 ml Ethylacetat extrahiert. Nach Evaporation wurden 1,07 g Produkt erhalten. Das Produkt 12-Keto-UDCS wurde mittels DC und NMR analysiert und bestätigt. Die 3alpha-HSDH wurde analog zur Herstellung von 7ß-HSDH, jedoch mit dem Plasmid pET22b+ hergestellt und ohne weitere Aufreinigung verwendet.

Schema 3: Schematische Darstellung von Reduktion von 3,12-Diketo-7β-CS durch die 3α-HSDH. Die FDH regeneriert den Cofaktor NADH.

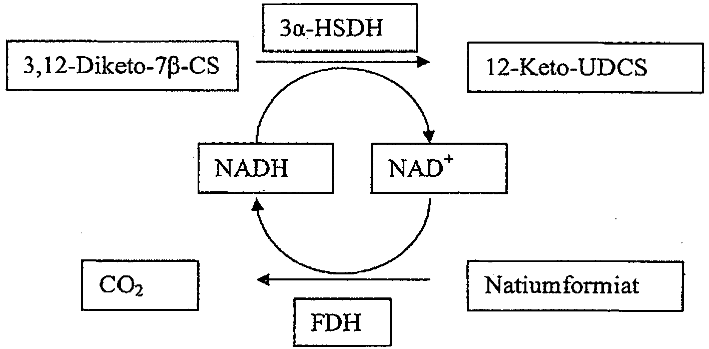

### Umsetzungsbeispiel 4: Chemische Umsetzung von CS zu DHCS

In einem 2000 I Rührwerksbehälter werden 1320 I Eisessig vorgelegt und darin 110 kg (260mol) Cholsäure (CS) gelöst. Zu dieser Lösung werden 422 I Natriumhypochlorit Lösung M/50180-EP

(2,3 molar) bei 20 bis 40 °C zudosiert und die Reaktionslösung anschließend noch mindestens 1 Stunde zur Vervollständigung der Reaktion nachgerührt. Die Dehydocholsäure (DHCS)wird in einer Ausbeute von 100 kg (90%) durch Zentrifugieren isoliert.

### Umsetzungsbeispiel 5: Chemische Umsetzung von 12-Keto-UDCS zu UDCS

105g (0,258mol) 12-Keto-UDCS werden in 384ml Triethylenglycol, 52,2g (1,304 mol) Natriumhydroxid und 75,95ml (1,563 mol) Hydrazinhydrat gelöst und langsam auf 180°C erwärmt. Dabei bildet sich das Hydrazon, das ab 160°C unter Abspaltung von Stickstoff in die UDCS umwandelt. Die Reaktion wird zur Vervollständigung der Umsetzung 8 Stunden bei 180°C gehalten. Die Reaktionsmischung wird unter 100°C gekühlt, mit 1500 ml Wasser versetzt. Anschließend durch Ansäuern mit Salzsäure die UDCS ausgefällt. Das Produkt wird in einer Ausbeute von 96,2 g bis 99,2 g (bis 95% - 98%) erhalten.

### Herstellungsbeispiel 2: Klonierung, Expression und Reinigung 7β-HSDH aus Collinsella aerofaciens ATCC 25986 in präparativem Maßstab sowie weitere Charakterisierung des Enzyms

### a) Klonierung und Herstellung eines Expressionskontruktes

Das für 7β-HSDH kodierende Gen wurde wiederum aus der genomischen DNA durch PCR und unter Verwendung von Primem, wie oben für Herstellungsbeispiel 1 beschrieben, amplifiziert:
Das PCR-Produkt wurde wiederum wie oben beschrieben gereinigt und mit den Restriktionsendonukleasen Ndel und HindIII verdaut. Das verdaute PCR-Produkt wurde wiederum gereinigt und in den pET-28a(+)-Vektor unter Verwendung der T4-Ligase kloniert, um einen Expressionsvektor zu erzeugen. Das resultierende Expressionskonstrukt wurde anschließend in *E. coli* DH5α-Zellen transformiert. Das zu erwartende Protein sollte 20 Aminosäurereste umfassendes ein Signalpeptid und einen N-terminalen 6xHis-Tag sowie eine Thrombin-Schnittstelle aufweisen. Die Sequenz der insertierten DNA wurde durch Sequenzierung überprüft.

### b) Überexpression und Reinigung von 7β-HSDH

E. *coli* BL21(DE3) wurde mit dem Expressionskonstrukt transformiert. Dazu wurde der das Expressionskonstrukt enthaltende *E*. *coli* BL21(DE3)-Stamm in LB-Medium (2 × 400 ml in 2 Liter-Schüttelflaschen) enthaltend 30 µg/ml Kanamycin vermehrt. Die Zellen wurden durch M/50180-EP
Zentrifugation (10,000×g, 15 min, 4°C) geerntet. Das Pellet wurde in 20 ml Phosphatpuffer (50 mM, pH 8, enthaltend 0.1 mM PMSF) resuspendiert. Die Zellen wurden unter konstanter Kühlung durch einminütige Ultraschallbehandlung (40 W Leistung, 40% Arbeitsintervall und 1 min Pause) unter Verwendung eines Ultraschallgeräts Sonifier 250 (Branson, Deutschland) aufgeschlossen. Der Aufschluss wurde dreimal wiederholt. Das Zeflextrakt wurde zentrifugiert (22,000×g, 20 min, 4°C). Der Überstand wurde auf eine Talon-Säule (Clontech, USA), äquilibriert mit Beladungspuffer (50 mM Kaliumphosphat, 300 mM NaCl, pH 8) beladen. Das Verfahren wurde bei 24°C durchgeführt. Nicht gebundenes Material wurde durch Waschen der Säule mit Beladungspuffer (3 Säulenvolumina) ausgewaschen. Schwach bindendes Protein wurde durch Waschen mit Waschpuffer (20 mM Imidazol im Beladungspuffer; 3 Säulenvolumina) entfernt. Das His-Tag-7β-HSDH-Protein wurde mit Elutionspuffer (200 mM Imidazol im Beladungspuffer) eluiert. Das Eluat wurde in einem Dialyseschlauch mit einer molekularen Ausschlussgrenze von 5 kDa (Sigma, USA) in 2 Liter Kaliumphosphatpuffer (50 mM, pH 8) bei 4°C über Nacht dialysiert. Schließlich wurde die Probe in einen neuen Schlauch überführt und bei -20°C zur weiteren Analyse gelagert. Die Proteinkonzentration wurde unter Verwendung eines BCA Testkits (Thermo, USA) nach Herstellerangaben bestimmt. Außerdem wurde die Probe durch 12.5%ige SDS-PAGE und Färben mit Coomassie Brilliant Blue analysiert. Die Reinheit des Proteins wurde densitometrisch mit Hilfe von Scion Image Beta 4.0.2 (Scion, USA) bestimmt.

### c) Gelfiltration

Gelfiltration wurde auf einem Pharmacia ÄKTA-Protein-Reinigungssystem durchgeführt, um das Molekulargewicht von 7β-HSDH zu bestimmen. Das gereinigte Enzym wurde auf eine Sephadex G-200-Säule aufgetragen, welche zuvor mit 50 mM Tris-HCl (pH 8), enthaltend 200 mM Natriumchlorid, äquilibriert worden war. Das Protein wurde mit dem gleichen Puffer bei einer Flussrate von 1 ml/min eluiert. Das Molekulargewicht von 7β-HSDH wurde durch Vergleich seines Elutionsvolumens mit demjenigen von Proteinstandards (Serum Albumin (66 kDa), α-Amylase aus *Aspergillus oryzae* (52 kDa), Trypsin aus Schweinepankreas (24 kDa) und Lysozym aus Hühnerei (14.4 kDa)) bestimmt.

### d) Enzymtest und kinetische Analyse

Das Reaktionsgemisch für den Enzymtest enthielt in einem Gesamtvolumen von 1 ml 50 µmol Kaliumphosphat (pH 8), 0.1 µmol NAD(P)H oder NAD(P)⁺, Substrate und Protein. Das Reaktionsgemisch war in Küvetten mit einer Lichtpfadlänge von 1 cm enthalten. Die 7β-HSDH-Aktivität wurde durch Aufzeichnung der Veränderung in der NAD(P)H-Konzentration über die Extinktion bei 340 nm mit Hilfe eines Spektrophotometers (Ultraspec 3000, Pharmacia Biotech, Großbritannien) bestimmt. Die Enzymaktivitäten wurden als Enzymeinheiten (U, d.h. µmol/min) unter Verwendung des molaren Extinktionskoeffizienten von 6,22 mM⁻¹ × cm⁻¹ bei 25°C bestimmt. Mehrere unterschiedliche Messungen mit den Variablen Substrat, Koenzym, Konzentration, pH-Wert, Puffer und Inkubationstemperatur wurden durchgeführt. Die kinetischen Konstanten wurden unter Verwendung von Standardmethoden bestimmt.

### e) Biotransformation von 7-keto-Lithocholsäure durch die 7β-HSDH

Die Umwandlung von 7-Keto-LCS durch 7β-HSDH wurde durchgeführt, um die biochemische Funktion von 7β-HSDH zu verifizieren. 0.4 g 7-Keto-LCS wurden in 10 ml Kaliumphosphatpuffer (50 mM, pH 8) suspendiert und der pH-Wert wurde auf pH 8 durch Zugabe von 2 M Natriumhydroxid eingestellt. 0.2 ml Isopropanol, 100 U 7β-HSDH und 80 U Alkoholdehydrogenase (ADH-TE) aus *Thermoanaerobacter ethanolicus* (freundliche Zuwendung von Frau Dr. K. Momoi, ITB Universität, Stuttgart) und 1 µmol NADP⁺ wurden zugesetzt. Der gleiche Puffer wurde zugesetzt, um ein gesamtes Reaktionsvolumen von 20 ml zu erhalten. Das Reaktionsgemisch wurde bei 24°C inkubiert und 24 Stunden gerührt. Dabei wurde NADPH mit Hilfe der ADH über die Oxidation von 2-Propanol regeneriert. Das Produkt wurde mit 1 ml 2 M Salzsäure angesäuert und 5 × mit 5 ml Ethylacetat extrahiert. Die organische Lösung wurde anschließend destilliert.

### f) Chromatographische Produktbestimmung

Eine HPLC-Analyse wurde auf einer Säule vom Typ Purospher® STAR RP-18 (Hitbar® RT 125-4 Pre-Packed Column, Purospher® STAR RP-18 endcapped, Merck, Deutschland), versehen mit einer Vorsäule des Typs LiChroCART® STAR RP18 (endcapped, Merck, Deutschland) auf einem HPLC-System LC20AD (Shimadzu, Japan) bei einer Flussrate von 1 ml/min durchgeführt. Die mobile Phase bestand aus zwei Eluenten. Eluent A enthielt Acetonitril und Eluent B destilliertes Wasser (pH 2.6, eingestellt mit Orthophosphorsäure, 85%). Folgender Gradient wurde verwendet: A 35% (8 min) - 35%-43% (1% min⁻¹) - 43%-70% (1% min⁻¹) - 70% (5 min) - 70%-35% (17.5% min⁻¹) - 35% (5 min); eluent A 65% (8 min) - 65%-57% (1% min⁻¹) - 57%-30% (1% min⁻¹) - 30% (5 min) - 30%-65% (17.5% min⁻¹) - 65% (5 min). 20 µl Probe (1 mg/ml) wurden analysiert. Authentische UDCS, 7-Keto-LCS und CDCS wurden bei gleicher Konzentration als Standards verwendet. Die Aufzeichnung erfolgte durch UV-Detektion bei 200 nm.

### g) Sequenzalignment und phylogenetische Analyse

Multiple Sequenz-Alignments wurden unter Verwendung der Clustal X-Software (Thompson et al., 1997, Nucleic Acid Research 25: 4876-82) erzeugt und modifiziert unter Verwendung der Jalview-Software (Clamp et al., 2004, Bioinformatics 20:426-7). Der phylogenetische Stammbaum wurde unter Verwendung des Programms TreeView 1.6.6 (Roderic 2001, http://taxonomy.zoology.gla.ac.uk/rod/rod.html) erzeugt.

### h) Versuchsergebnisse:

### 1. Identifizierung der 7β-HSDH-Aktivität in einer präparativen Biotransformation

Um die Funktion des Enzyms zu bestätigen, wurde eine Biotransformation von 7-Keto-LCS im 10 ml-Maßstab durchgeführt, wobei das isolierte Enzym in Kombination mit ADH zur Regeneration von NADPH unter Verwendung von 2-Propanol, wie bereits beschrieben, eingesetzt wurde. Die HPLC-Analyse ergab, dass UDCS das einzige von dem Enzym produzierte Reaktionsprodukt (90% Umsatz) war. CDCS (Retentionszeit 19,4 min) wurde im Reaktionsgemisch nicht nachgewiesen. Das Ergebnis zeigt, dass das Enzym eine NADPH-abhängige 7β-HSDH darstellt und zur selektiven Reduktion der 7-Carbonyl-Gruppe von 7-Keto-LCS in eine 7β-Hydroxy-Gruppe befähigt ist.
Retentionszeit UDCS: 15,5 min
Retentionszeit 7-keto-LCS: 18,3 min

### 2. Reinigung und Gelfiltration

Nach Klonierung des 7β-HSDH-Gens aus *Collinsella aerofaciens* DSM 3979 in den Expressionsvektor pET28a(+) und anschließende Überexpression erhielt man ein am N-terminus mit einem His-Tag versehenes Fusionsprotein mit einer 7β-HSDH-Ausbeute von 332.5 mg (5828 U) pro Literkultur. Das mit dem His-Tag versehene 7β-HSDH wurde in einer Stufe mit Hilfe einer Immobilisierte-Metallionen-Affinitätschromatographie gereinigt (Reinheit >90%, Ausbeute 76%, vgl. Fig. 2.), Die Hauptbanden der Bahnen 1 und 2 repräsentieren das erwartete Expressionsprodukt bei 30 kDa, welches dem aus der Aminosäuresequenz des Gens abgeleiteten vorhergesagten Molekulargewicht entspricht. Durch Gelfiltration ermittelt man jedoch für die 7β-HSDH ein Molekulargewicht von 56.1 kDa. Dies belegt die dimere Beschaffenheit der 7β-HSDH aus *Collinsella aerofaciens* DSM 3979.

### 3. Sequenzalignments

Die Aminosäuresequenz der erfindungsgemäßen 7β-HSDH wurde mit bekannten HSDH-Sequenzen verglichen (vgl. Fig. 3). Die beobachtete Sequenzähnlichkeit weist darauf hin, dass das erfindungsgemäße Enzym zur Familie der kurzkettigen Dehydrogenasen (SDR) gehört. Es ist bekannt, dass SDRs sehr niedrige Homologie und Sequenzidentität aufweisen (Jomvall, H., B. Persson, M. Krook, S. Atrian, R. Gonzalez-Duarte, J. Jeffery, and D. Ghosh. 1995. Short-chain dehydrogenases/reductases (SDR). Biochemistry 34:6003-13 und Persson, B., M. Krook, and H. Jomvall. 1991. Characteristics of short-chain alcohol dehydrogenases and related enzymes. Eur J Biochem 200:537-43). Das Sequenzalignment zeigt jedoch deutlich die konservierten Domänen in der SDR Primärstruktur. Das N-terminale Motiv Gly-X-X-X-Gly-X-Gly (entsprechend Gly-41, Gly-45 and Gly-47, Nummerierung entsprechend dem Alignment) entspricht dem charakteristischen Dinukleotid-Bindungsmotiv der SDR-Superfamilie. Darüber hinaus sind drei stark konservierte Reste Ser-177, Tri-190 und Lys-194 (Nummerierung gemäß Alignment) zu erkennen, die der katalytischen Triade der SDR-Enzyme entsprechen.

### 4. Phylogenetische Analyse

Der evolutionäre Stammbaum basierend auf dem Alignment von Fig. 3 ist in Fig. 4 dargestellt. 7α-HSDH aus *Clostridium sordellii, Brucella melitensis* und *Escherichia coli* gehören zur gleichen Untergruppe. Die beiden 3α-HSDHs zeigen eine ausgeprägtere Verwandtschaft als andere HSDHs. Interessanterweise ist die prokaryotische 7β-HSDH mit der tierischen 11β-HSDH-Untergruppe, umfassend *Cavia porcellus, Homo sapiens* und *Mus musulus,* verwandt.

### 5. Kinetische Konstanten

Kinetische Gleichgewichtsanalysen wurden durchgeführt, um die absoluten Werte für Vₘₐₓ und K_{M} für UDCS, 7-Keto-LCS, DHCS, NADP⁺ und NADPH mit Hilfe von Lineweaver-Burk-Plots zu bestimmen. In folgender Tabelle sind sämtliche kinetische Daten für die getesteten Substrate und Coenzyme, die aus Substratsättigungskurven und reziproke Auftragungen erhalten wurden, zusammengefasst. Die Vₘₐₓ, K_{M} and k_{cat}-Werte für sämtliche Substrate und Coenzyme liegen im gleichen Bereich, wohingegen der K_{M}-Wert für DHCS signifikant höher lag als bei den anderen Substraten, eventuell bedingt durch die geringe Löslichkeit in Wasser. Das Enzym ist NADPH-abhängig und kinetische Konstanten für NAD⁺ und NADH konnten aufgrund der sehr geringen Aktivität nicht bestimmt werden.

**Tabelle 1**

| Zusammenfassung von kinetischen Konstanten für 7β-HSDH aus *Collinsella aerofaciens* DSM 3979. | | | |
|---|---|---|---|
| | K_{M} (µM) | Vₘₐₓ (U/mg Protein)^{b)} | k_{cat}(1 µmol/(µmol×min)) |
| NADP⁺ | 5.32 | 30.58 | 944.95 |
| NADPH | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| NAD⁺ | -^{a)} | - | Spuren |
| NADH | - | - | Spuren |

| | | | |
|---|---|---|---|
| ^{a)} konnten aufgrund der sehr geringen Aktivität nicht bestimmt werden ^{b)} 1 U = 1 µmol/min | | | |

### 6. pH-Optimum

Weiterhin wurde die 7β-HSDH-Aktivität für verschiedene Substrate in Abhängigkeit vom pH-Wert mit gereinigtem Enzym bestimmt (vgl. Fig. 5). Für die Oxidation von UDCS mit 7β-HSDH wurde eine optimale Aktivität im Bereich von pH 9 bis 10 bei allmählichem Abfall auf der sauren Seite beobachtet. Im Gegensatz dazu ergab sich für die Reduktion von DHCS und 7-keto-LCS durch die 7β-HSDH) eine optimale Aktivität im Bereich von pH 4 bis 6 mit einem scharfen Abfall auf der sauren Seite und einem allmählichen Abfall auf der alkalischen Seite. Unterschiedliche Puffer haben nur einen geringen Einfluss auf die Aktivität der 7β-HSDH bei gleichem pH-Wert.

### 7. Thermische Stabilität

Die erfindungsgemäße NADP-abhängige 7β-HSDH zeigt folgende s Stabilitätsverhalten: Nach 400 min war die Aktivität bei 30°C etwa 30% niedriger als bei 23°C. Das Enzym war bei 30°C nach 1500 min vollständig inaktiviert, während bei 23°C und 1500 min die verbleibende Aktivität 20% betrug. Kein signifikanter Aktivitätsverlust wurde während der Lagerung bei -20°C in Kaliumphosphatpuffer (50 mM, pH 8) über einen Zeitraum von einigen Monaten nach mehrmaligem Einfrieren und Auftauen beobachtet.

**Zuordnung von SEQ ID NOs:**

| SEQ ID NO: | Beschreibung | Typ |
|---|---|---|
| 1 | 7ß-HSDH | NS |
| 2 | 7ß-HSDH | AS |
| 3 | Primer | NS |
| 4 | Primer | NS |
| 5 | 3α-HSDH (C. testosteroni) | NS |
| 6 | 3α-HSDH (C. testosteroni) | AS |
| 7 | 3α-HSDH (R. norvegicus) | NS |
| 8 | 3α-HSDH (R. norvegicus) | AS |

| | | |
|---|---|---|
| AS = Aminosäuresequenz NS= Nukleinsäuresequenz | | |

Auf die Offenbarung der hierin erwähnten Druckschriften wird ausdrücklich Bezug genommen.

## Patentansprüche

1. 7β-HSDH umfassend eine Aminosäuresequenz gemäß SEQ ID NO: 2 oder eine davon abgeleitete Sequenz mit einem Identitätsgrad von wenigstens 80% zu dieser Sequenz, mit einem Molekulargewicht, bestimmt durch Gelfiltration, von etwa 53 bis 60 kDa und welche zumindest die stereospezifische Reduktion eines 7-Ketosteroids zum korrespondierenden 7β-Hydroxysteroid katalysiert.

2. 7ß-HSDH nach Anspruch 1 mit einem Molekulargewicht, bestimmt durch Gelfiltration, von etwa 56,1 kDa.

3. Verfahren zur enzymatischen Synthese von 7β-Hydroxysteroiden, wobei man das korrespondierende 7-Ketosteroid in Gegenwart einer 1ß-HSDH gemäß der Definition in einem der Ansprüche 1 und 2 umsetzt, und wenigstens ein gebildetes Reduktionsprodukt aus dem Reaktionsansatz gegebenenfalls isoliert.

4. Verfahren nach Anspruch 3, wobei das zu reduzierende Ketosteroid ausgewählt ist unter Dehydrocholsäure (DHCS),
7-Keto-lithocholsäure (7-Keto-LCS),
7,12-Diketo-lithocholsäure (7,12-Diketo-LCS) und
den Derivaten davon, wie insbesondere einem Salz, Amid oder Alkylester der Säure.

5. Verfahren nach einem der Ansprüche 3 und 4, wobei die Reduktion in Gegenwart von NAD(P)H erfolgt.

6. Verfahren zur enzymatischen Oxidation von 7ß-Hydroxysteroiden, wobei man das Hydroxysteroid in Gegenwart einer 7ß-HSDH gemäß der Definition in einem der Ansprüche 1 und 2 umsetzt, und ein gebildetes Oxidationsprodukt aus dem Reaktionsansatz gegebenenfalls isoliert.

7. Verfahren nach Anspruch 6, wobei das 7ß-Hydroxysteroid 3,12-Diketo-7ß-CS oder ein Derivat davon, wie insbesondere ein Salz, Amid oder Alkylester, ist.

8. Verfahren nach einem der Ansprüche 6 und 7, wobei die Oxidation in Gegenwart von NAD(P)⁺ erfolgt.

9. Verfahren nach einem der Ansprüche 5 und 8, wobei die verbrauchten Redox-Äquivalente elektrochemisch oder enzymatisch regeneriert werden.

10. Verfahren nach Anspruch 9, wobei verbrauchtes NAD(P)H durch Kopplung mit einem NAD(P)H-regenerierenden Enzym ausgewählt unter einer NAD(P)H Dehydrogenase und insbesondere einer Alkoholdehydrogenase (ADH), regeneriert wird.

11. Verfahren nach Anspruch 10, wobei das NAD(P)H-regenerierende Enzym ausgewählt ist unter natürlichen oder rekombinanten, isolierten oder angereicherten
a) Alkoholdehydrogenasen (EC 1.1.1.2) und
b) davon abgeleiteten funktionalen Äquivalenten

12. Verfahren zur Herstellung von Ursodesoxycholsäure (UDCS) der Formel (1) 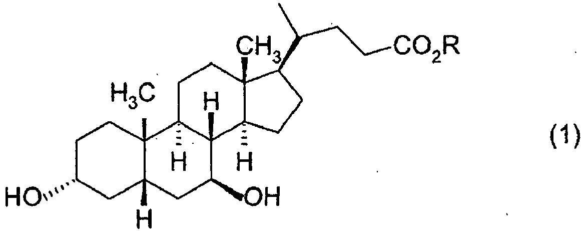 worin
R für Alkyl, H, ein Alkalimetallion oder N(R³)₄⁺steht, worin die Reste R³ gleich oder verschieden sind und für H oder Alkyl stehen,
wobei man
a) gegebenenfalls eine Cholsäure (CS) der Formel (2) 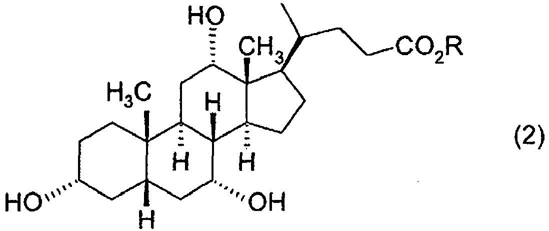 worin R die oben angegebenen Bedeutungen besitzt, zur Dehydrocholsäure (DHCS) der Formel (3) 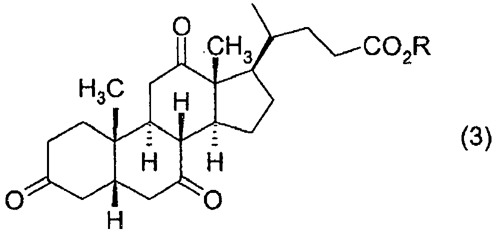 worin R die oben angegebenen Bedeutungen besitzt, chemisch oxidiert;
b) DHCS in Gegenwart wenigstens einer rekombinant hergestellten 7ß-HSDH gemäß der Definition in einem der Ansprüche 1 bis 2 zur 3,12-Diketo-7ß-cholansäure (3,12-Diketo-7ß-CS) der Formel (4) 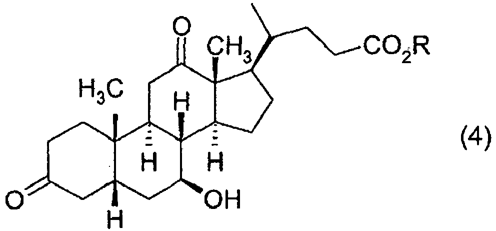 reduziert,
c) 3,12-Diketo-7ß-CS in Gegenwart wenigstens einer 3α-Hydroxysteroiddehydrogenase (3α-HSDH) zur korrespondierenden 12-Keto-ursodesoxycholsäure (12-Keto UDCS) der Formel (5) 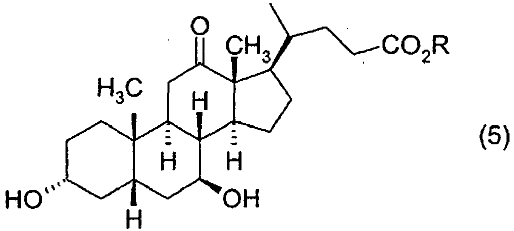 worin R die oben angebebenen Bedeutungen besitzt, reduziert und anschließend
d) 12-Keto-UDCS der Formel (5) chemisch zu UDCS reduziert; und
e) das Reaktionsprodukt gegebenenfalls weiter aufreinigt.

13. Verfahren nach Anspruch 12, wobei die Schritte b) und/oder c) mit einem Cofaktorregenerierungsschritt gekoppelt sind.

14. Verfahren nach Anspruch 13, wobei
Schritt b) mit einem Cofaktorregenerierungsschritt gekoppelt ist, bei welchem NADPH durch Alkoholdehydrogenase (ADH) unter Verbrauch eines Opferalkohols regeneriert wird; und/oder Schritt c) mit einem Cofaktorregenerierungsschritt gekoppelt ist, bei welchem NADH durch Formiatdehydrogenase (FDH) unter Verbrauch von Formiat regeneriert wird; oder bei welchem NADPH durch Alkoholdehydrogenase (ADH) unter Verbrauch eines Opferalkohols regeneriert wird.

## Claims

1. 7β-HSDH comprising an amino acid sequence according to SEQ ID No.: 2 or a sequence derived therefrom having an identity degree of at least 80% to this sequence, having a molecular weight, determined by gel filtration, of about 53 to 60 kDa and which at least catalyses the stereospecific reduction of a 7-ketosteroid to form the corresponding 7β-hydroxysteroid.

2. 7β-HSDH according to claim 1 having a molecular weight, determined by gel filtration, of about 56.1 kDa.

3. Method for the enzymatic synthesis of 7β-hydroxysteroids, wherein the corresponding 7-ketosteroid is converted in the presence of a 7β-HSDH according to the definition in any of claims 1 and 2, and at least one reduction product formed is optionally isolated from the reaction batch.

4. Method according to claim 3, wherein the ketosteroid to be reduced is selected from dehydrocholic acid (DHCA),
7-keto-lithocholic acid (7-keto-LCA),
7,12-diketo-lithocholic acid (7,12-diketo-LCA) and
the derivatives thereof, such as in particular a salt, amide or alkyl ester of the acid.

5. Method according to any of claims 3 and 4, wherein the reduction takes place in the presence of NAD(P)H.

6. Method for the enzymatic oxidation of 7β-hydroxysteroids, wherein the hydroxysteroid is converted in the presence of a 7β-HSDH according to the definition in any of claims 1 and 2, and an oxidation product formed is optionally isolated from the reaction batch.

7. Method according to claim 6, wherein the 7β-hydroxysteroid is 3,12-diketo-7β-CA or a derivative thereof, such as in particular a salt, amide or alkyl ester.

8. Method according to any of claims 6 and 7, wherein the oxidation takes place in the presence of NAD(P)⁺.

9. Method according to any of claims 5 and 8, wherein the redox equivalents consumed are electrochemically or enzymatically regenerated.

10. Method according to claim 9, wherein consumed NAD(P)H is regenerated by coupling with an NAD(P)H-regenerating enzyme selected from an NAD(P)H dehydrogenase and in particular an alcohol dehydrogenase (ADH).

11. Method according to claim 10, wherein the NAD(P)H-regenerating enzyme is selected from natural or recombinant, isolated or enriched
a) alcohol dehydrogenases (EC 1.1.1.2) and
b) functional equivalents derived therefrom.

12. Method for the production of ursodesoxycholic acid (UDCA) of formula (1) 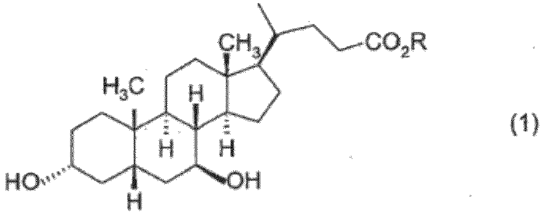 wherein
R stands for alkyl, H, an alkali metal ion or N(R³)₄⁺, wherein the groups R³ are the same or different and stand for H or alkyl,
Wherein
a) a cholic acid (CA) of formula (2) 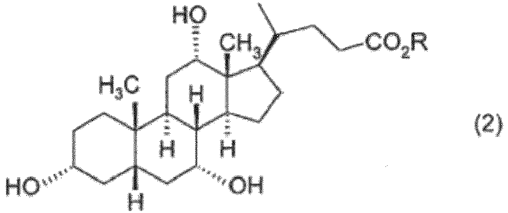 wherein R has the aforesaid meanings, is optionally chemically oxidized to form the dehydrocholic acid (DHCA) of formula (3) 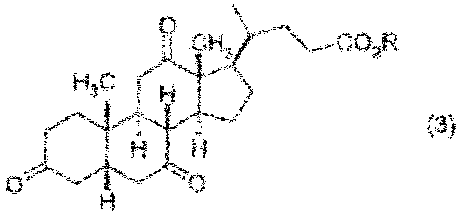 wherein R has the aforesaid meanings;
b) DHCA is reduced in the presence of at least one recombinantly prepared 7β-HSDH according to the definition in any of claims 1 to 2 to form the 3,12-diketo-7β-cholanic acid (3,12-diketo-7β-CA) of formula (4) 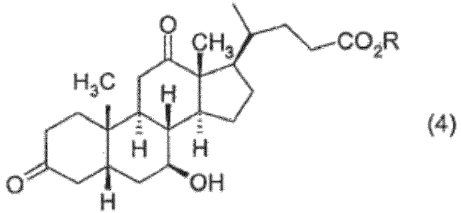
c) 3,12-diketo-7β-CS is reduced in the presence of at least one 3α-hydroxysteroid dehydrogenase (3α-HSDH) to form the corresponding 12-keto-ursodesoxycholic acid (12-keto UDCA) of formula (5) 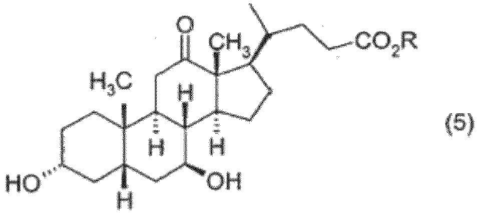 wherein R has the aforesaid meanings, and then
d) 12-keto-UDCA of formula (5) is chemically reduced to form UDCA; and
e) the reaction product is optionally further purified.

13. Method according to claim 12, wherein steps b) and/or c) are coupled with a cofactor regeneration step.

14. Method according to claim 13, wherein step b) is coupled with a cofactor regeneration step, in which NADPH is regenerated by alcohol dehydrogenase (ADH) with consumption of a sacrificial alcohol, and/or step c) is coupled with a cofactor regeneration step, in which NADH is regenerated by formate dehydrogenase (FDH) with consumption of formate, or in which NADPH is regenerated by alcohol dehydrogenase (ADH)with consumption of a sacrificial alcohol.

## Revendications

1. 7β-HSDH comprenant une séquence d'acides aminés selon SEQ ID NO:2 ou une séquence dérivée de celle-ci ayant un degré d'identité d'au moins 80 % avec cette séquence, ayant un poids moléculaire, déterminé par filtration sur gel, d'environ 53 à 60 kDa et qui catalyse au moins la réduction stéréospécifique d'un 7-cétostéroïde en le 7β-hydroxystéroïde correspondant.

2. 7β-HSDH selon la revendication 1 ayant un poids moléculaire, déterminé par filtration sur gel, d'environ 56,1 kDa.

3. Procédé pour la synthèse enzymatique de 7β-hydroxy-stéroïdes où l'on fait réagir le 7-cétostéroïde correspondant en présence d'une 7β-HSDH selon la définition dans l'une des revendications 1 et 2, et on isole éventuellement au moins un produit de réduction formé à partir de la masse réactionnelle.

4. Procédé selon la revendication 3 où le cétostéroïde à réduire est choisi parmi l'acide déshydrocholique (ADHC), l'acide 7-cétolithocholique (A 7-céto-LC), l'acide 7,12-dicéto-lithocholique (A 7,12-dicéto-LC) et leurs dérivés, comme, en particulier, un sel, un amide ou un ester d'alkyle de l'acide.

5. Procédé selon l'une des revendications 3 et 4 où la réduction a lieu en présence de NAD(P)H.

6. Procédé pour l'oxydation enzymatique de 7β-hydroxystéroïdes où l'on fait réagir l'hydroxystéroïde en présence d'une 7β-HSDH selon la définition dans l'une des revendications 1 et 2, et on isole éventuellement un produit d'oxydation formé à partir de la masse réactionnelle.

7. Procédé selon la revendication 6 où le 7β-hydroxystéroïde est le A 3,12-dicéto-7β-C ou un dérivé de celui-ci, comme, en particulier, un sel, un amide ou un ester d'alkyle.

8. Procédé selon l'une des revendications 6 et 7 où l'oxydation a lieu en présence de NAD(P)⁺.

9. Procédé selon l'une des revendications 5 et 8 où les équivalents redox consommés sont régénérés par voie électrochimique ou enzymatique.

10. Procédé selon la revendication 9 où le NAD(P)H consommé est régénéré par couplage avec une enzyme régénérant le NAD(P)H choisie parmi une NAD(P)H déshydrogénase et en particulier une alcool déshydrogénase (ADH).

11. Procédé selon la revendication 10 où l'enzyme régénérant le NAD(P)H est choisie parmi
a) les alcools déshydrogénases (EC 1.1.1.2) naturelles ou recombinantes, isolées ou concentrées, et
b) les équivalents fonctionnels dérivés de celles-ci.

12. Procédé pour la production d'acide ursodésoxycholique (AUDC) de formule (1) 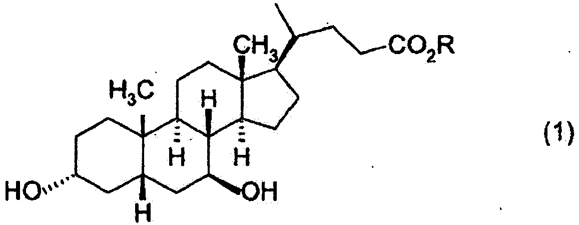 où
R représente alkyle, H, un ion de métal alcalin ou N(R³)₄⁺, où les groupements R³ sont identiques ou différents et représentent H ou alkyle, où
a) éventuellement on oxyde chimiquement un acide cholique (AC) de formule (2) 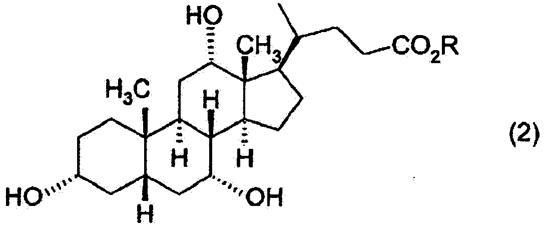 où R possède les significations indiquées ci-dessus, en l'acide déshydrocholique (ADHC) de formule (3) 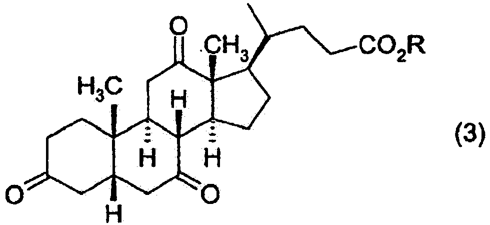 où R possède les significations indiquées ci-dessus;
b) on réduit le ADHC en présence d'au moins une 7β-HSDH selon la définition dans l'une des revendications 1 à 2 produite de manière recombinante en l'acide 3,12-dicéto-7β-cholanique (A 3,12-dicéto-7β-C) de formule (4) 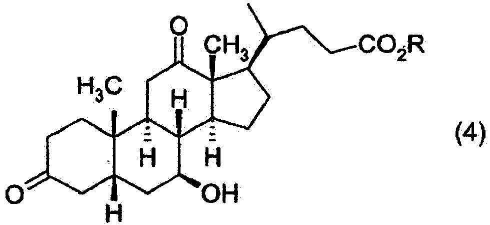
c) on réduit le A 3,12-dicéto-7β-C en présence d'au moins une 3α-hydroxystéroïde déshydrogénase (3α-HSDH) en l'acide 12-cétoursodésoxycholique (A 12-céto-UDC) correspondant de formule (5) 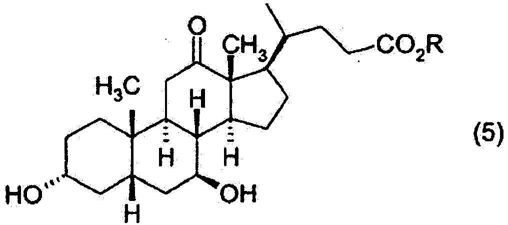 où R possède les significations indiquées ci-dessus, puis
d) on réduit le A 12-céto-UDC de formule (5) chimiquement en AUDC; et
e) éventuellement on purifie encore le produit réactionnel.

13. Procédé selon la revendication 12 où les étapes b) et/ou c) sont couplées avec une étape de régénération de cofacteur.

14. Procédé selon la revendication 13 où l'étape b) est couplée avec une étape de régénération de cofacteur dans laquelle le NADPH est régénéré par une alcool déshydrogénase (ADH) avec consommation d'un alcool sacrificiel; et/ou l'étape c) est couplée avec une étape de régénération de cofacteur dans laquelle le NADH est régénéré par une formiate déshydrogénase (FDH) avec consommation de formiate; ou bien dans laquelle le NADPH est régénéré par une alcool déshydrogénase (ADH) avec consommation d'un alcool sacrificiel.


### SEQUENCE LISTING

<110> PharmaZell GmbH
<120> Neuartige 7beta-Hydroxysteroiddehydrogenasen und deren Verwendung
<130> M/50180-PCT
<160> 8
<170> PatentIn version 3.3
<210> 1
   <211> 792
   <212> DNA
   <213> Collinsella aerofaciens
<220>
   <221> CDS
   <222> (1)..(792)
<400> 1
   atg aac ctg agg gag aag tac ggt gag tgg ggc ctg atc ctg ggc gcg     48
   Met Asn Leu Arg Glu Lys Tyr Gly Glu Trp Gly Leu Ile Leu Gly Ala
   1               5                   10                  15
   acc gag ggc gtc ggc aag gcg ttc tgc gag aag atc gcc gcc ggc ggc     96
   Thr Glu Gly Val Gly Lys Ala Phe Cys Glu Lys Ile Ala Ala Gly Gly
               20                  25                  30
   atg aac gtc gtc atg gtc ggc cgt cgc gag gag aag ctg aac gtg ctc    144
   Met Asn Val Val Met Val Gly Arg Arg Glu Glu Lys Leu Asn Val Leu
           35                  40                  45
   gca ggc gag atc cgc gag acc tac ggc gtg gag acc aag gtc gtg cgc    192
   Ala Gly Glu Ile Arg Glu Thr Tyr Gly Val Glu Thr Lys Val Val Arg
       50                  55                  60
   gcc gac ttt agc cag ccc ggc gct gcc gag acc gtc ttc gcc gcg acc    240
   Ala Asp Phe Ser Gln Pro Gly Ala Ala Glu Thr Val Phe Ala Ala Thr
   65                  70                  75                  80
   gag ggc ctg gac atg ggc ttc atg agc tac gtg gcc tgc ctg cac agc    288
   Glu Gly Leu Asp Met Gly Phe Met Ser Tyr Val Ala Cys Leu His Ser
                   85                  90                  95
   ttc ggt aag atc cag gac acc ccc tgg gag aag cac gag gcc atg atc    336
   Phe Gly Lys Ile Gln Asp Thr Pro Trp Glu Lys His Glu Ala Met Ile
               100                 105                 110
   aac gtc aac gtc gtg acc ttc ctc aag tgc ttc cac cac tac atg cgg    384
   Asn Val Asn Val Val Thr Phe Leu Lys Cys Phe His His Tyr Met Arg
           115                 120                 125
   atc ttt gcc gcc cag gac cgc ggc gcc gtg atc aac gtc tcg tcg atg    432
   Ile Phe Ala Ala Gln Asp Arg Gly Ala Val Ile Asn Val Ser Ser Met
       130                 135                 140
   acc ggc atc agc tcc agc ccc tgg aac ggc cag tac ggc gcg ggc aag    480
   Thr Gly Ile Ser Ser Ser Pro Trp Asn Gly Gln Tyr Gly Ala Gly Lys
   145                 150                 155                 160
   gcc ttc atc ctc aag atg acc gag gcc gtg gcc tgc gag tgc gag ggc    528
   Ala Phe Ile Leu Lys Met Thr Glu Ala Val Ala Cys Glu Cys Glu Gly
                   165                 170                 175
   acc ggc gtc gac gtc gag gtc atc acc ctc ggc acc acc cta acc ccc        576
   Thr Gly Val Asp Val Glu Val Ile Thr Leu Gly Thr Thr Leu Thr Pro
               180                 185                 190
   agc ctg ctg tcc aac ctc ccc ggc ggc ccg cag ggc gag gcc gtc atg        624
   Ser Leu Leu Ser Asn Leu Pro Gly Gly Pro Gln Gly Glu Ala Val Met
           195                 200                 205
   aag atc gcc ctc acc ccc gag gag tgc gtt gac gag gcc ttt gag aag        672
   Lys Ile Ala Leu Thr Pro Glu Glu Cys Val Asp Glu Ala Phe Glu Lys
       210                 215                 220
   ctg ggt aag gag ctc tcc gtc atc gcc ggc cag cgc aac aag gac tcc        720
   Leu Gly Lys Glu Leu Ser Val Ile Ala Gly Gln Arg Asn Lys Asp Ser
   225                 230                 235                 240
   gtc cac gac tgg aag gca aac cac acc gag gac gag tac atc cgc tac        768
   Val His Asp Trp Lys Ala Asn His Thr Glu Asp Glu Tyr Ile Arg Tyr
                   245                 250                 255
   atg ggg tcg ttc tac cgc gac tag                                        792
   Met Gly Ser Phe Tyr Arg Asp
               260
<210> 2
   <211> 263
   <212> PRT
   <213> Collinsella aerofaciens
<400> 2
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Gly Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
   130 135 140
   Thr Gly Ile Ser Ser Ser Pro Trp Asn Gly Gln Tyr Gly Ala Gly Lys
   145 150 155 160
   Ala Phe Ile Leu Lys Met Thr Glu Ala Val Ala Cys Glu Cys Glu Gly
   165 170 175
   Thr Gly Val Asp Val Glu Val Ile Thr Leu Gly Thr Thr Leu Thr Pro
   180 185 190
   Ser Leu Leu Ser Asn Leu Pro Gly Gly Pro Gln Gly Glu Ala Val Met
   195 200 205
   Lys Ile Ala Leu Thr Pro Glu Glu Cys Val Asp Glu Ala Phe Glu Lys
   210 215 220
   Leu Gly Lys Glu Leu Ser Val Ile Ala Gly Gln Arg Asn Lys Asp Ser
   225 230 235 240
   Val His Asp Trp Lys Ala Asn His Thr Glu Asp Glu Tyr Ile Arg Tyr
   245 250 255
   Met Gly Ser Phe Tyr Arg Asp
   260
<210> 3
   <211> 31
   <212> DNA
   <213> artificial
<220>
   <223> PCR Primer
<400> 3
   gggaattcca tatgaacctg agggagaagt a 31
<210> 4
   <211> 27
   <212> DNA
   <213> Artificial
<220>
   <223> PCR Primer
<400> 4
   cccaagcttc tagtcgcggt agaacga 27
<210> 5
   <211> 774
   <212> DNA
   <213> Comamonas testosteroni
<220>
   <221> CDS
   <222> (1)..(774)
<400> 5
   atg tcc atc atc gtg ata agc ggc tgc gcc acc ggc att ggt gcg gct        48
   acg cgc aag gtc ctg gag gcg gcc ggt cac cag atc gta ggc atc gat        96
   ata cgc gat gcg gaa gtg att gcc gat ctc tcg acg gcc gaa ggt cga       144
   aag cag gcg att gcc gat gta ctg gcg aag tgc agc aag ggc atg gac       192
   ggc ctg gtg ctg tgc gcc ggc ctg gga ccg cag acc aag gtg ctt ggc       240
   aat gtg gtt tcg gtc aat tat ttt ggc gcg acc gag ctg atg gat gcc       288
   ttt ttg cca gcg ctg aaa aaa ggc cat cag ccc gca gcc gtc gtc atc       336
   tcg tcc gtg gct tcc gcg cat ctg gct ttt gac aag aac cca ctg gcg       384
   ctg gca ctg gaa gcc ggc gag gaa gcc aag gcc cgc gcc att gtc gaa       432
   cat gcg gga gag cag ggc gga aat ctg gcc tat gcg ggc agc aag aat       480
   gct ttg acg gtg gct gtg cgc aaa cgc gcc gcc gcc tgg ggc gag gct       528
   ggc gtg cgc ctg aac acc atc gcc ccc ggt gca acc gag act ccc ttg       576
   ctg cag gcg ggc ctg cag gac ccg cgc tat ggc gaa tcc att gcc aag       624
   ttc gtt cct ccc atg ggc cgc cgt gcc gag ccg tcc gag atg gcg tcg       672
   gtc atc gcc ttt ttg atg agc ccg gcc gca agc tat gtg cat ggc gcg       720
   cag atc gtc att gat ggc ggc att gat gcg gtg atg cgc ccg aca cag        768
   ttc tga                                                                774
<210> 6
   <211> 257
   <212> PRT
   <213> Comamonas testosteroni
<400> 6
<210> 7
   <211> 969
   <212> DNA
   <213> Rattus norvegicus
<220>
   <221> CDS
   <222> (1)..(969)
<400> 7
   atg gat tcc ata tct ctg cgt gta gca cta aat gat ggt aac ttc att      48
   Met Asp Ser Ile Ser Leu Arg Val Ala Leu Asn Asp Gly Asn Phe Ile
   1               5                   10                  15
   cct gta ctg ggg ttt gga acc act gtg cct gag aag gtt gct aag gat      96
   Pro Val Leu Gly Phe Gly Thr Thr Val Pro Glu Lys Val Ala Lys Asp
               20                  25                  30
   gaa gtt atc aag gct act aaa ata gct ata gat aat gga ttc cgc cat      144
   Glu Val Ile Lys Ala Thr Lys Ile Ala Ile Asp Asn Gly Phe Arg His
           35                  40                  45
   ttt gac tct gct tat ttg tac gaa gta gaa gag gaa gtg ggc caa gcc      192
   Phe Asp Ser Ala Tyr Leu Tyr Glu Val Glu Glu Glu Val Gly Gln Ala
       50                  55                  60
   att aga agc aag att gaa gac ggc act gtg aag aga gaa gat ata ttc      240
   Ile Arg Ser Lys Ile Glu Asp Gly Thr Val Lys Arg Glu Asp Ile Phe
   65                  70                  75                  80
   tat act tca aag ctt tgg agc act ttc cat aga cca gag ctg gtc cga      288
   Tyr Thr Ser Lys Leu Trp Ser Thr Phe His Arg Pro Glu Leu Val Arg
                   85                  90                  95
   act tgc ttg gaa aag aca ctg aaa agc act caa ctg gac tat gtg gat      336
   Thr Cys Leu Glu Lys Thr Leu Lys Ser Thr Gln Leu Asp Tyr Val Asp
               100                 105                 110
   ctt tat att att cat ttc cca atg gct ttg cag cct gga gat ata ttt      384
   Leu Tyr Ile Ile His Phe Pro Met Ala Leu Gln Pro Gly Asp Ile Phe
           115                 120                 125
   ttc cca cga gat gag cat gga aaa cta ttg ttt gaa aca gtg gat atc      432
   Phe Pro Arg Asp Glu His Gly Lys Leu Leu Phe Glu Thr Val Asp Ile
       130                 135                 140
   tgt gac aca tgg gag gcc atg gaa aag tgt aag gat gca gga ttg gcc      480
   Cys Asp Thr Trp Glu Ala Met Glu Lys Cys Lys Asp Ala Gly Leu Ala
   145                 150                 155                 160
   aag tct att ggg gtg tcc aac ttt aac tgc agg cag ctg gag agg att      528
   Lys Ser Ile Gly Val Ser Asn Phe Asn Cys Arg Gln Leu Glu Arg Ile
                   165                 170                 175
   ctg aat aag cca ggg ctc aaa tac aag cct gtg tgc aac cag gtg gaa      576
   Leu Asn Lys Pro Gly Leu Lys Tyr Lys Pro Val Cys Asn Gln Val Glu
               180                 185                 190
   tgt cac ctt tat ctc aac cag agc aaa atg ctg gac tat tgt aag tca      624
   Cys His Leu Tyr Leu Asn Gln Ser Lys Met Leu Asp Tyr Cys Lys Ser
           195                 200                 205
   aaa gac atc att ctg gtt tcc tac tgc acg ctg gga agt tca cga gac      672
   Lys Asp Ile Ile Leu Val Ser Tyr Cys Thr Leu Gly Ser Ser Arg Asp
       210                 215                 220
   aaa aca tgg gtg gat cag aaa agt cca gtt ctc cta gat gat cca gtt      720
   Lys Thr Trp Val Asp Gln Lys Ser Pro Val Leu Leu Asp Asp Pro Val
   225                 230                 235                 240
   ctt tgt gcc ata gca aag aag tac aag caa acc cca gcc cta gtt gcc      768
   Leu Cys Ala Ile Ala Lys Lys Tyr Lys Gln Thr Pro Ala Leu Val Ala
                   245                 250                 255
   ctt cgc tac cag ctg cag cgt ggg gtt gtg ccc ctg atc agg agt ttc      816
   Leu Arg Tyr Gln Leu Gln Arg Gly Val Val Pro Leu Ile Arg Ser Phe
               260                 265                 270
   aac gcg aag cgg atc aaa gag cta aca cag gtt ttt gaa ttc cag ttg      864
   Asn Ala Lys Arg Ile Lys Glu Leu Thr Gln Val Phe Glu Phe Gln Leu
           275                 280                 285
   gct tca gag gac atg aaa gcc ctg gat ggc ttg aac aga aat ttc aga      912
   Ala Ser Glu Asp Met Lys Ala Leu Asp Gly Leu Asn Arg Asn Phe Arg
       290                 295                 300
   tac aac aat gca aaa tat ttt gat gac cat ccc aat cat cca ttt act      960
   Tyr Asn Asn Ala Lys Tyr Phe Asp Asp His Pro Asn His Pro Phe Thr
   305                 310                 315                 320
   gat gaa tag                                                          969
   Asp Glu
<210> 8
   <211> 322
   <212> PRT
   <213> Rattus norvegicus
<400> 8
   Met Asp Ser Ile Ser Leu Arg Val Ala Leu Asn Asp Gly Asn Phe Ile
   1               5                   10                  15
   Pro Val Leu Gly Phe Gly Thr Thr Val Pro Glu Lys Val Ala Lys Asp
               20                  25                  30
   Glu Val Ile Lys Ala Thr Lys Ile Ala Ile Asp Asn Gly Phe Arg His
           35                  40                  45
   Phe Asp Ser Ala Tyr Leu Tyr Glu Val Glu Glu Glu Val Gly Gln Ala
       50                  55                  60
   Ile Arg Ser Lys Ile Glu Asp Gly Thr Val Lys Arg Glu Asp Ile Phe
   65                  70                  75                  80
   Tyr Thr Ser Lys Leu Trp Ser Thr Phe His Arg Pro Glu Leu Val Arg
                   85                  90                  95
   Thr Cys Leu Glu Lys Thr Leu Lys Ser Thr Gln Leu Asp Tyr Val Asp
               100                 105                 110
   Leu Tyr Ile Ile His Phe Pro Met Ala Leu Gln Pro Gly Asp Ile Phe
           115                 120                 125
   Phe Pro Arg Asp Glu His Gly Lys Leu Leu Phe Glu Thr Val Asp Ile
       130                 135                 140
   Cys Asp Thr Trp Glu Ala Met Glu Lys Cys Lys Asp Ala Gly Leu Ala
   145                 150                 155                 160
   Lys Ser Ile Gly Val Ser Asn Phe Asn Cys Arg Gln Leu Glu Arg Ile
                   165                 170                 175
   Leu Asn Lys Pro Gly Leu Lys Tyr Lys Pro Val Cys Asn Gln Val Glu
               180                 185                 190
   Cys His Leu Tyr Leu Asn Gln Ser Lys Met Leu Asp Tyr Cys Lys Ser
           195                 200                 205
   Lys Asp Ile Ile Leu Val Ser Tyr Cys Thr Leu Gly Ser Ser Arg Asp
       210                 215                 220
   Lys Thr Trp Val Asp Gln Lys Ser Pro Val Leu Leu Asp Asp Pro Val
   225                 230                 235                 240
   Leu Cys Ala Ile Ala Lys Lys Tyr Lys Gln Thr Pro Ala Leu Val Ala
                   245                 250                 255
   Leu Arg Tyr Gln Leu Gln Arg Gly Val Val Pro Leu Ile Arg Ser Phe
               260                 265                 270
   Asn Ala Lys Arg Ile Lys Glu Leu Thr Gln Val Phe Glu Phe Gln Leu
           275                 280                 285
   Ala Ser Glu Asp Met Lys Ala Leu Asp Gly Leu Asn Arg Asn Phe Arg
   290 295 300
   Tyr Asn Asn Ala Lys Tyr Phe Asp Asp His Pro Asn His Pro Phe Thr
   305 310 315 320
   Asp Glu